# EUROPEAN PATENT SPECIFICATION

(11) **EP 4 041 281 B1**
(45) Date of publication and mention of the grant of the patent: **26.11.2025**
(21) Application number: 20793900.0
(22) Date of filing: 02.10.2020
(51) Int. Cl.: A61K 38/18, C07K 16/00, A61P 9/00, A61P 9/02

(54) **USE OF GDF15 FOR TREATING CARDIOMETABOLIC SYNDROME AND OTHER CONDITIONS**
VERWENDUNG VON GDF15 ZUR BEHANDLUNG VON KARDIOMETABOLISCHEM SYNDROM UND ANDERE KRANKHEITEN
UTILISATION DE GDF15 POUR LE TRAITEMENT DU SYNDRÔME CARDIOMÉTABOLIQUE ET D'AUTRES MALADIES

(30) Priority: 04.10.2019 US 201962910661 P
(43) Date of publication of application: 17.08.2022
(73) Proprietor: Amgen Inc., Thousand Oaks, California 91320 (US)
(72) Inventor: ELLISON, Murielle Marie V., Thousand Oaks, California 91320-1799 (US); HALE, Clarence H., Thousand Oaks, California 91320-1799 (US); LUO, Xin, Thousand Oaks, California 91320-1799 (US); STOLINA, Marina, Thousand Oaks, California 91320-1799 (US)
(74) Representative: Grünecker Patent- und Rechtsanwälte PartG mbB
(86) International application number: PCT/US2020/053884
(87) International publication number: WO 2021/067655

(56) References cited:
- EP-A1- 2 098 244
- WO-A1-2013/113008
- KAI C WOLLERT ET AL: "Growth Differentiation Factor 15 in Heart Failure: An Update", CURRENT HEART FAILURE REPORTS, CURRENT SCIENCE INC, NEW YORK, vol. 9, no. 4, 9 September 2012 (2012-09-09), pages 337 - 345, XP035134728, ISSN: 1546-9549, DOI: 10.1007/S11897-012-0113-9
- SHANNON E. MULLICAN ET AL: "Uniting GDF15 and GFRAL: Therapeutic Opportunities in Obesity and Beyond", TRENDS IN ENDOCRINOLOGY AND METABOLISM, vol. 29, no. 8, 1 June 2018 (2018-06-01), US, pages 560 - 570, XP055600819, ISSN: 1043-2760, DOI: 10.1016/j.tem.2018.05.002
- LEITE SARA ET AL: "Arterial Remodeling and Dysfunction in the ZSF1 Rat Model of Heart Failure With Preserved Ejection Fraction", CIRCULATION. HEART FAILURE (PRINT), vol. 12, no. 7, 1 July 2019 (2019-07-01), US, XP055766389, ISSN: 1941-3289, DOI: 10.1161/CIRCHEARTFAILURE.118.005596
- REDDY SUKKA SANTOSH ET AL: "Cilostazol ameliorates heart failure with preserved ejection fraction and diastolic dysfunction in obese and non-obese hypertensive mice", JOURNAL OF MOLECULAR AND CELLULAR CARDIOLOGY, ACADEMIC PRESS, GB, vol. 123, 20 August 2018 (2018-08-20), pages 46 - 57, XP085505525, ISSN: 0022-2828, DOI: 10.1016/J.YJMCC.2018.08.017
- STOLINA MARINA ET AL: "The evolving systemic biomarker milieu in obese ZSF1 rat model of human cardiometabolic syndrome: Characterization of the model and cardioprotective effect of GDF15", PLOS ONE, vol. 15, no. 8, 17 August 2020 (2020-08-17), pages e0231234, XP055766386, DOI: 10.1371/journal.pone.0231234

## Description

### BACKGROUND

Growth differentiation factor 15 (GDF15), also referred to as macrophage inhibitory cytokine 1 (MIC1) (Bootcov MR, 1997, Proc Natl Acad Sci 94:11514-9), placental bone morphogenetic factor (PLAB) (Hromas R 1997, Biochim Biophys Acta. 1354:40-4), placental transforming growth factor beta (PTGFB) (Lawton LN 1997, Gene. 203:17-26), prostate derived factor (PDF) (Paralkar VM 1998, J Biol Chem. 273:13760-7), and nonsteroidal anti-inflammatory drug-activated gene (NAG-1) (Back SJ 2001, J Biol Chem. 276: 33384-92), is a secreted protein that circulates in plasma as an ~25 kDa homodimer. GDF15 binds to GDNF family receptor α-like (GFRAL) with high affinity. GDF15-induced cell signaling is believed to require the interaction of GFRAL with the coreceptor RET. GDF15 has been linked to multiple biological activities. Elevated GDF15 has been shown to be correlated with weight loss and administration of GDF15 has been shown to reduce food intake and body weight. WO2013/113008 discloses GDF15 polypeptides, constructs comprising GDF15, and mutants thereof.

Cardiometabolic syndrome (CMS), a combination of impaired metabolism (insulin resistance, impaired glucose tolerance, dyslipidemia), obesity, hypertension, renal insufficiency and cardiovascular diseases (CVD), is now recognized as a disease entity by the World Health Organization and the American Society of Endocrinology. Comorbidities, such as obesity and diabetes mellitus, are key factors of metabolic risk and are associated with the progressive left ventricular (LV) remodeling and dysfunction observed in heart failure with preserved ejection fraction (HFpEF). The incidence of HFpEF (currently 50% of heart failure) continues to rise, and its prognosis fails to improve partly due to the lack of a specific HFpEF therapy. Reddy et al, J. Mol. Cell. Cardiol., 2018 Oct:123:46-57 discloses that the Type III phosphodiesterase inhibitor Cilostazol ameliorates heart failure with preserved ejection fraction and diastolic dysfunction in obese and non-obese hypertensive mice.

An important step in the development of novel therapeutic agents against CMS is the establishment of a translational preclinical model, that represents a cluster of cardiometabolic disturbances.

Accordingly, there is a need for a translational preclinical model of CMS, as well as treatment therapies for treating CMS and other conditions, such as heart failure. The present disclosure meets these needs and provide related advantages.

### SUMMARY

Provided herein is a GDF15 molecule for use in a method of treating a heart condition in a subject, the method comprising administering the GDF15 molecule to the subject, wherein the heart condition is heart failure with preserved ejection fraction (HFpEF).

Further provided is a GDF15 molecule for use in a method of treating a subject with cardiometabolic syndrome, the method comprising administering the GDF15 molecule to the subject. In some embodiments, the subject is obese, has impaired metabolism, pancreatic dysfunction, hypertension, diastolic dysfunction, HFpEF, decreased exercise capacity, or renal dysfunction.

In some embodiments, after treatment with the GDF15 molecule, the subject has a decreased heart weight, improved exercise capacity, or change in the level of adiponectin, sE-selectin, sICAM, Myl3, osteopontin, TIMP1, VEGF, or vWF, after treatment with the GDF15 molecule. In some embodiments, the level of adiponectin is increased. In some embodiments, the level of se-selectin, sICAM, Myl3, osteopontin, TIMP1, VEGF, vWF, or any combination thereof is decreased. In yet other embodiments, the subject has decreased heart to brain weight ratio, left ventricle (LV) mass, cardiac output, stroke volume, or % ejection fraction echocardiographic, after treatment with the GDF15 molecule.

In some embodiments, the GDF15 molecule is a GDF15-Fc fusion protein. The fusion protein can comprise a GDF15 region joined to an Fc region. In some embodiments, the GDF15 region is joined to the Fc via a linker.

### BRIEF DESCRIPTION OF THE DRAWINGS

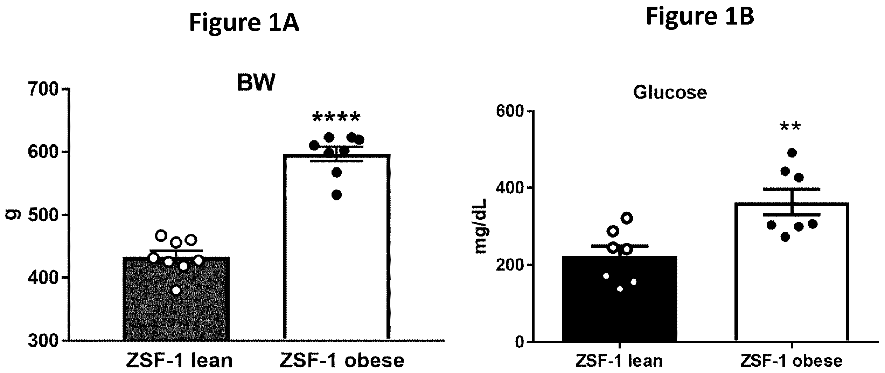

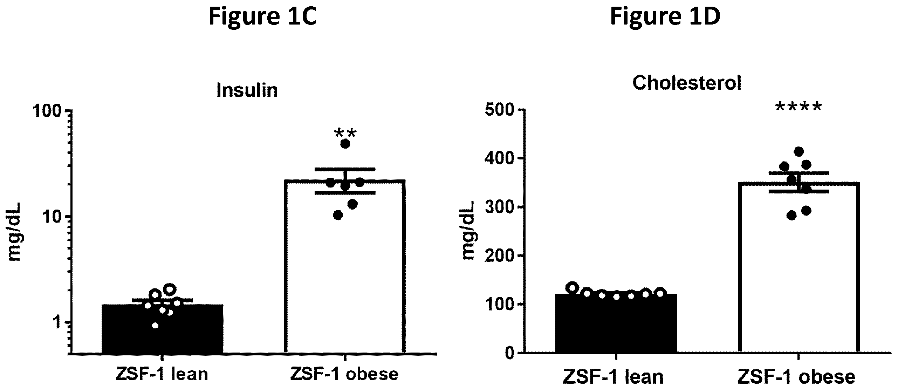

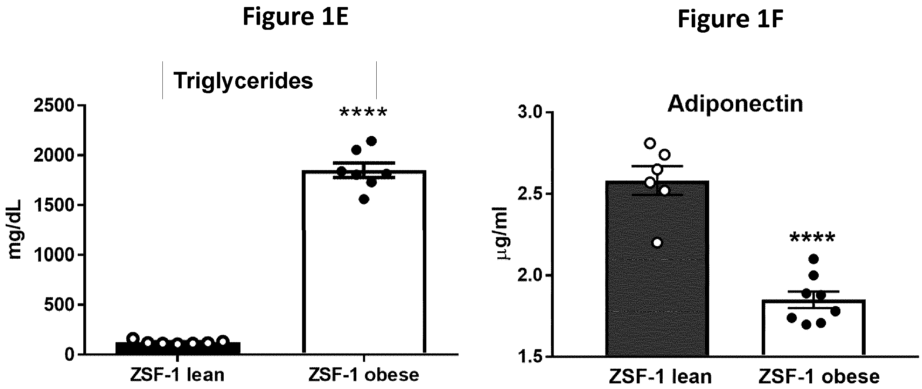

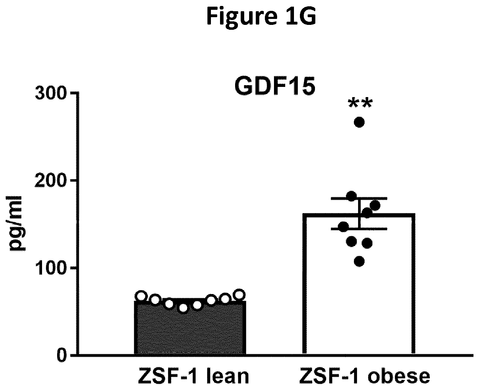

**Figure 1****.** ZSF1 obese male rats at the age of 20 weeks exhibit impaired metabolism by increased body weight (**Figure 1A**), elevated blood glucose (**Figure 1B**), insulin (**Figure 1C**), cholesterol (**Figure 1D**) and triglyceride levels (**Figure 1E**), statistically significant decrease in adiponectin (**Figure 1F**) and increase in GDF15 levels (**Figure 1G**). Stars (*) indicate significance (* p <0.05, ** p <0.01, *** p <0.001, **** p <0.0001), unpaired two-tailed t-test.

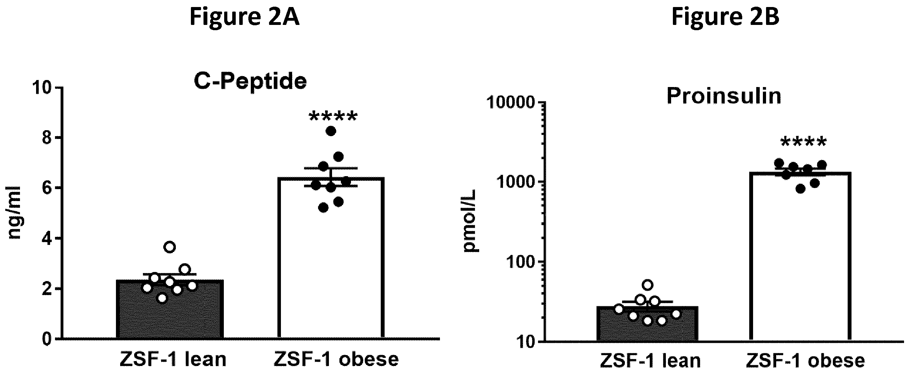

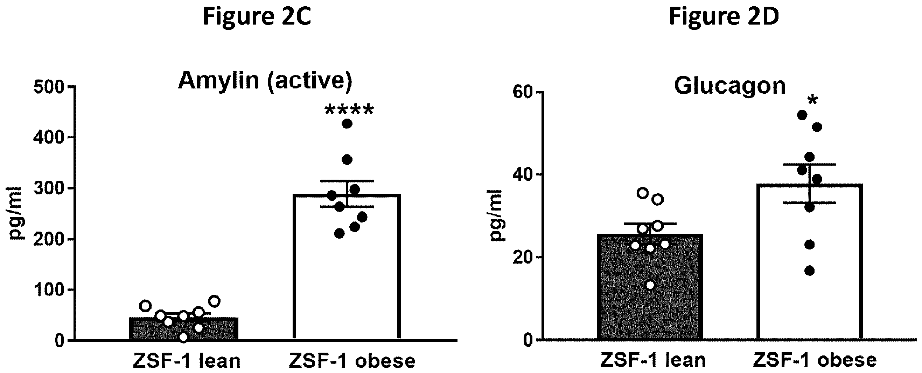

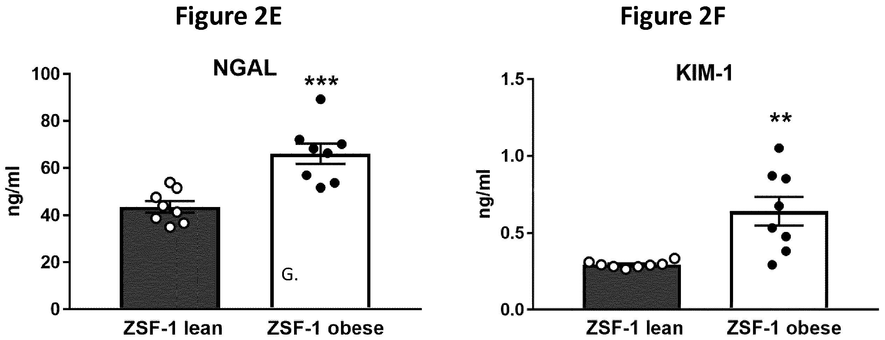

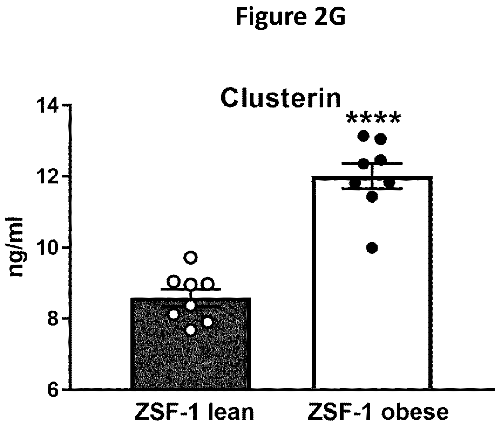

**Figure 2****.** ZSF1 obese rats demonstrate pancreatic disfunction as demonstrated with significant increase of C-peptide (**Figure 2A**), proinsulin (**Figure 2B**) active amylin (**Figure 2C**) and glucagon (**Figure 2D**). ZSF1 obese rats exhibit impaired renal function by increased serum levels of kidney injury markers NGAL (**Figure 2E**), KIM-1(**Figure 2F**) and clusterin (**Figure 2G**). Stars (*) indicate significance (* p <0.05, ** p <0.01, *** p <0.001, **** p <0.0001), unpaired two-tailed t-test.

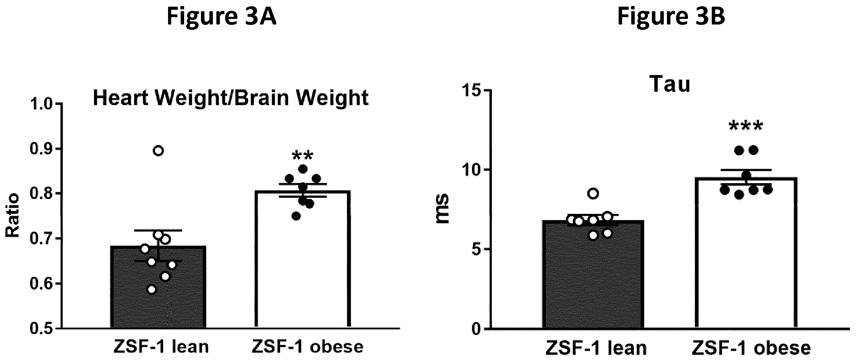

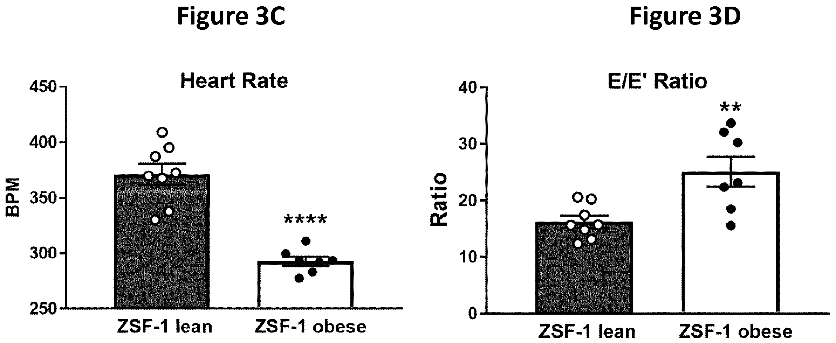

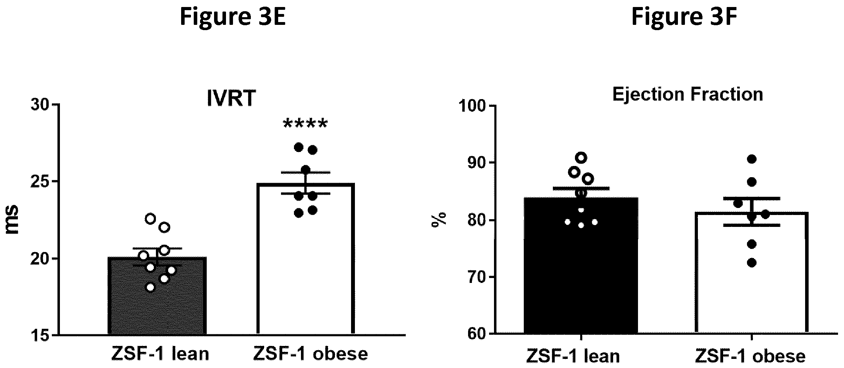

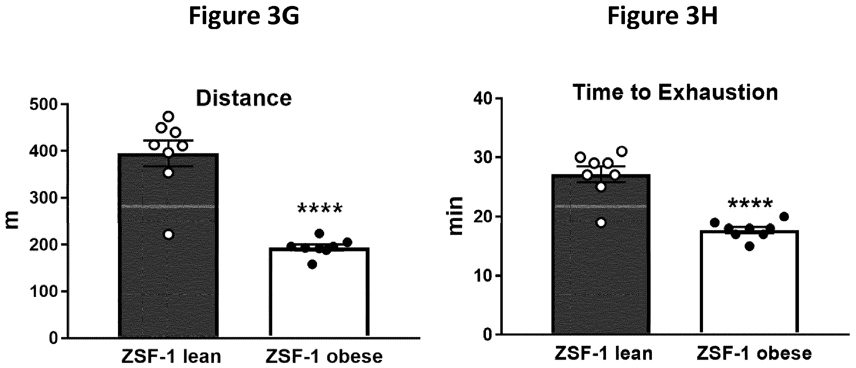

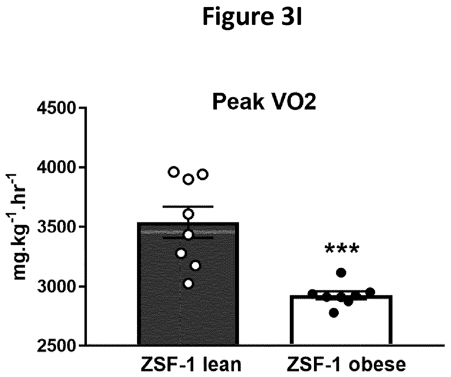

**Figure 3****.** ZSF1 obese rats exhibit diastolic dysfunction and decreased exercise capacity. The heart to brain weight ratio (by echocardiography) was significantly higher in ZSF1 obese rats (**Figure 3A**), invasive hemodynamic assessment provides evidence of diastolic dysfunction by increased *Tau* (**Figure 3B**) when compared to ZSF-1 lean rats. Echocardiography reveals that ZSF1 obese rats exhibit a decrease in heart rate (**Figure 3C**) and diastolic dysfunction by increase in the E/E' ratio (**Figure 3D**) and IVRT (**Figure 3E**), while %EF is not different from ZSF1 lean (**Figure 3F**) cohort. ZSF1 obese rats exhibit decreased exercise capacity as measure by distance (**Figure 3G**), time (**Figure 3H**), and peak VO2 (**Figure 3I**). Stars (*) indicate significance (* p <0.05, ** p <0.01, *** p <0.001, **** p <0.0001), unpaired two-tailed t-test.

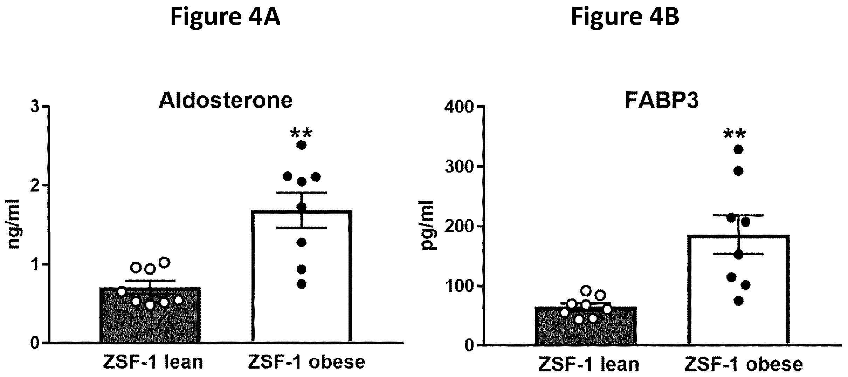

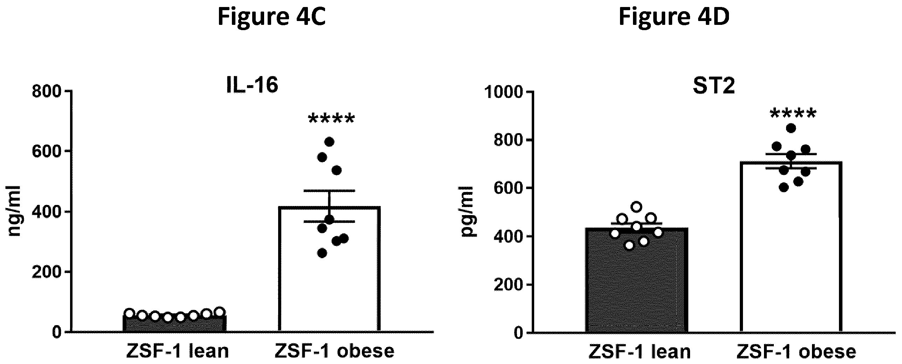

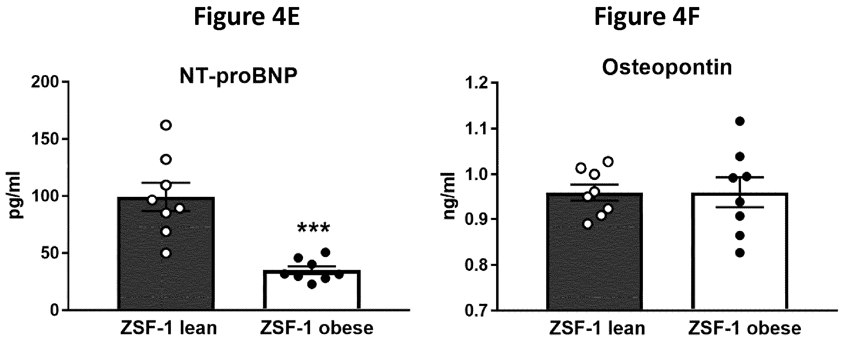

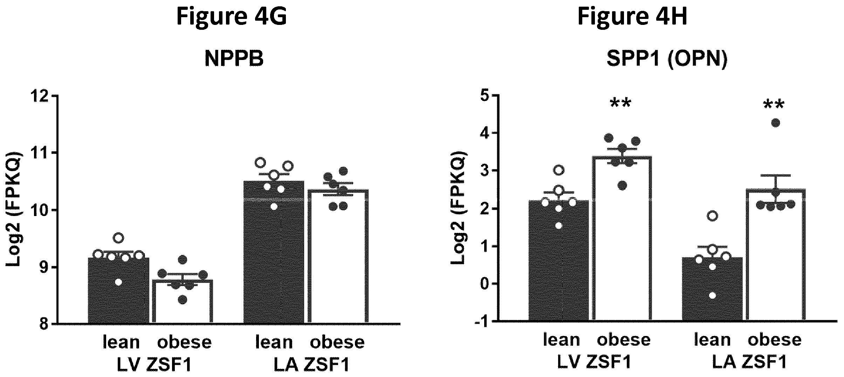

**Figure 4****.** Twenty-week-old ZSF1 obese male rats exhibit cardiovascular disfunction by increased blood levels of aldosterone (**Figure 4A**), FABP3 (**Figure 4B**), IL-16 (**Figure 4C**) and ST2 (**Figure 4D**). In ZSF1 obese rats, systemic concentration of NT-proBNP (**Figure 4E**) is significantly lower compared to the lean rats and osteopontin (**Figure 4F**) levels in circulation are not different between two groups of rats. The gene expression of NPPB (NT-proBNP protein, **Figure 4G**) in left heart was not different between ZSF1 obese and lean rats, whereas the local SPP1 (osteopontin) (**Figure 4H**) (expression in left atria and left ventricle was significantly elevated in ZSF1 obese rats. Stars (*) indicate significance (* p <0.05, ** p <0.01, *** p <0.001, **** p <0.0001), unpaired two-tailed t-test.

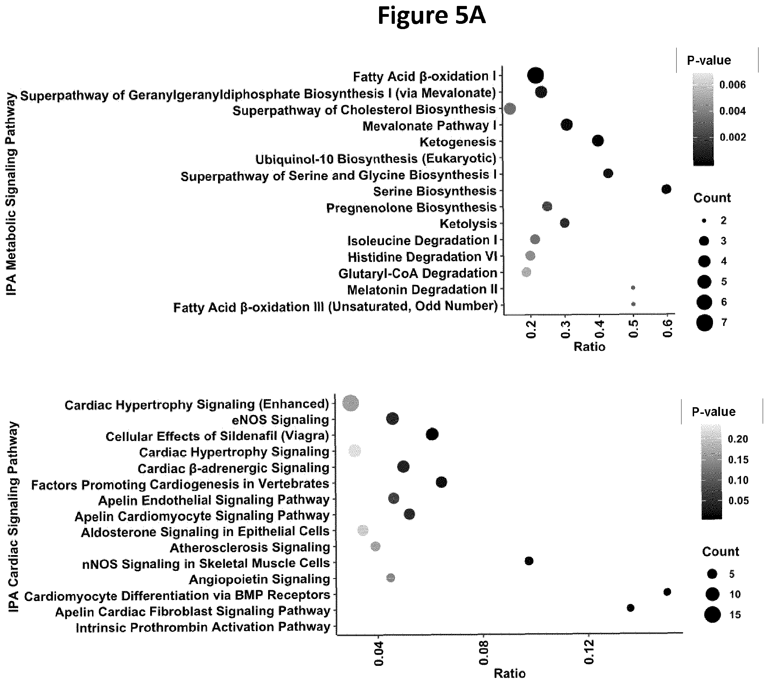

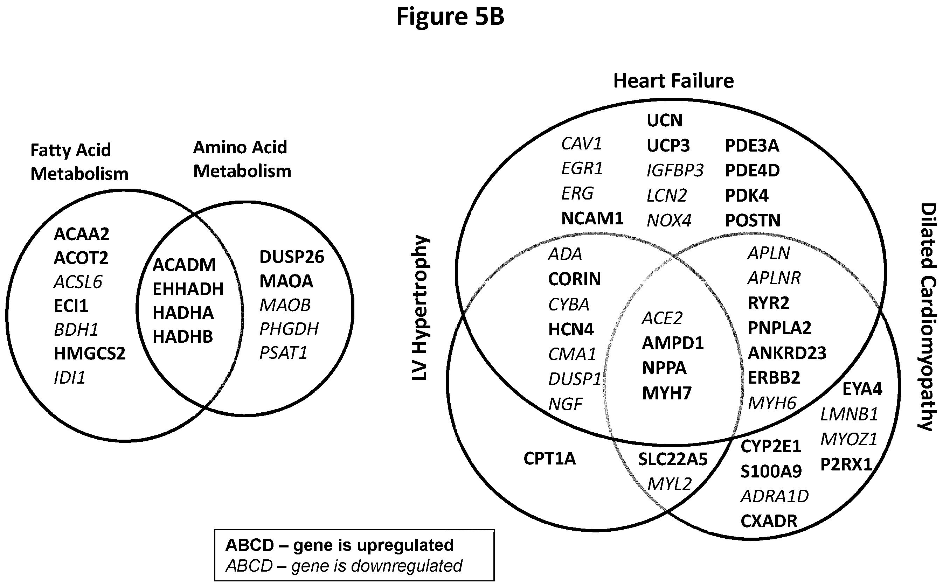

**Figure 5****.** The gene expression analysis of RNA extracted from left ventricle (LV) of twenty-week-old ZSF1 obese and lean male rats: the obesity-related gene expression changes in left ventricle included 267 significantly increased (FC>1.5, p<0.05) and 431 significantly decreased genes (FC<0.67, p<0.05), ), of which 56 increased genes and 48 decreased genes are heart abundant. Ingenuity pathway analysis (IPA) and MeSH term cardiac disease enrichment analysis revealed enriched metabolic and cardiac signaling pathways (**Figure 5A**) and overlapping genes in related pathways such as fatty acid metabolism, amino acid metabolism, or left ventricle (LV) hypertrophy, dilated cardiomyopathy, and heart failure (**Figure 5B**).

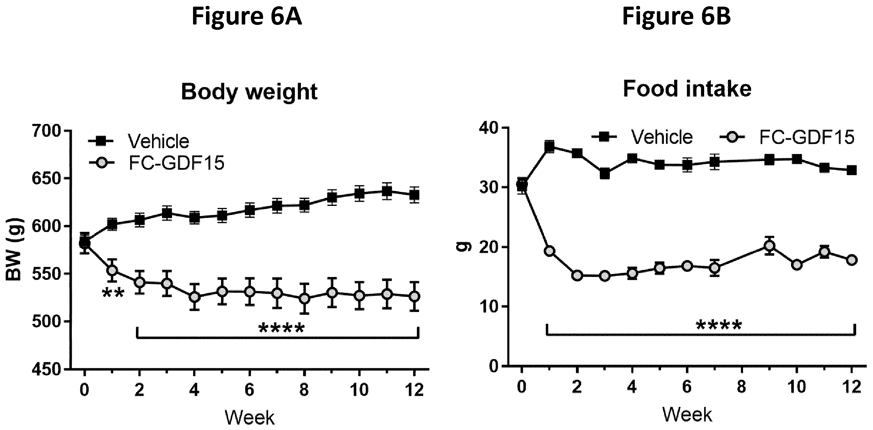

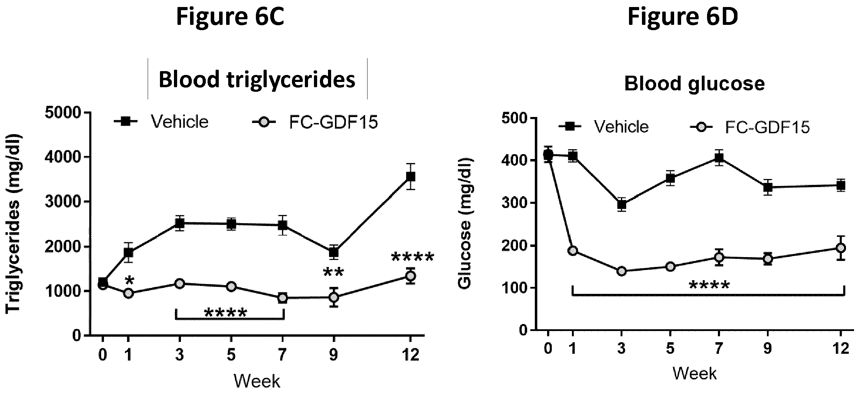

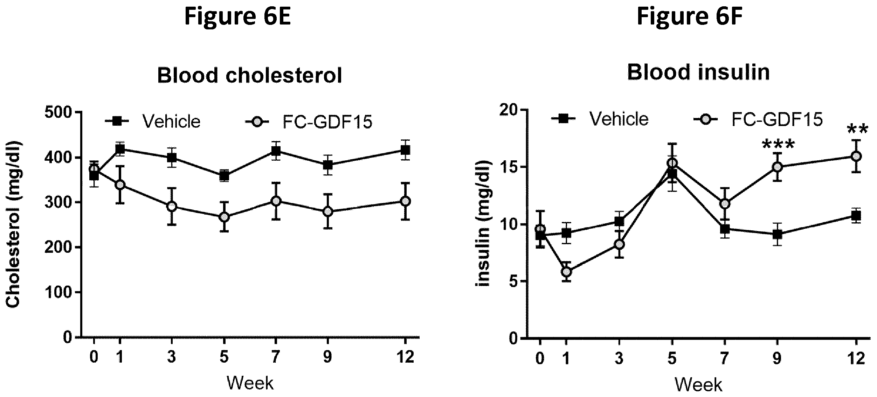

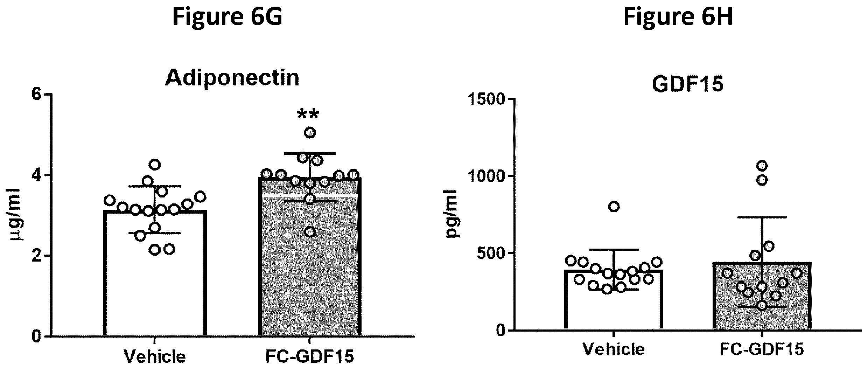

**Figure 6****.** Treatment of ZSF1 obese rat with FC-GDF15 resulted in statistically significant decrease in body weight (**Figure 6A**), food intake (**Figure 6B**), blood triglyceride (**Figure 6C**) and glucose levels (**Figure 6D**), and trend to decrease total cholesterol (**Figure 6E**). Blood insulin was significantly increased at week 9 and 12 in rats treated with GDF15 compared to vehicle treated rats (**Figure 6F**). Twelve weeks of FC-GDF15 treatment resulted in statistically significant increase in blood adiponectin (**Figure 6G**) and had no effect on endogenous rat GDF15 (**Figure 6H**) Stars (*) indicate significance (* p <0.05, ** p <0.01, *** p <0.001, **** p <0.0001), unpaired two-tailed t-test.

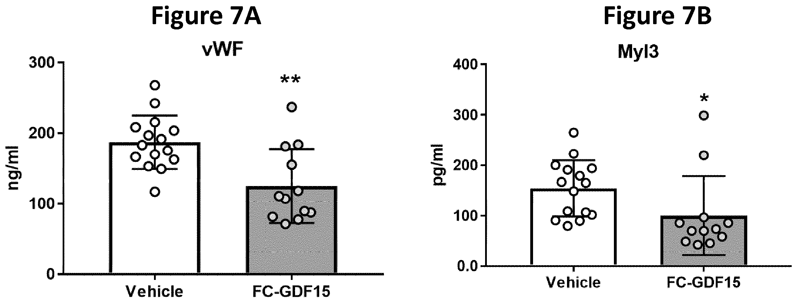

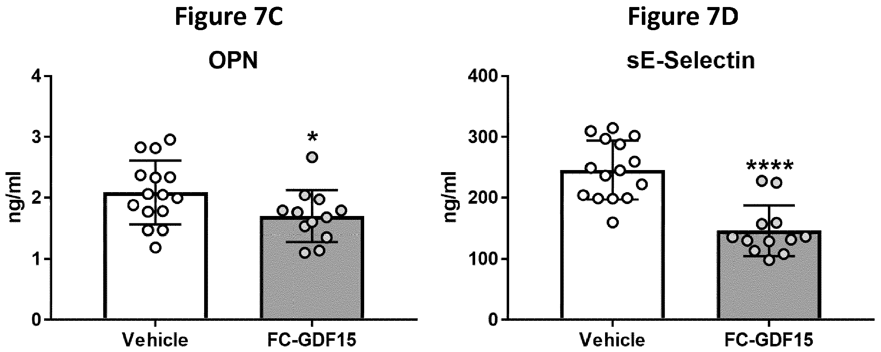

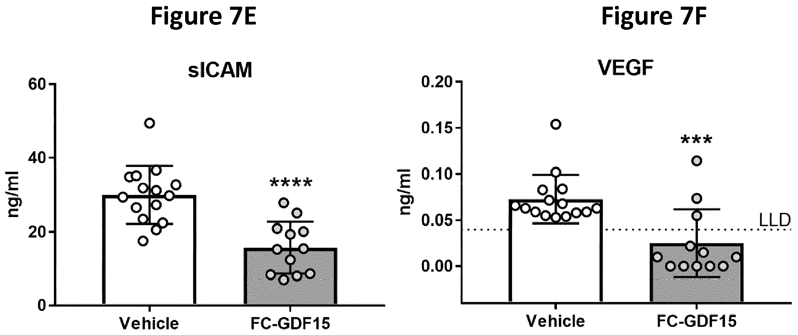

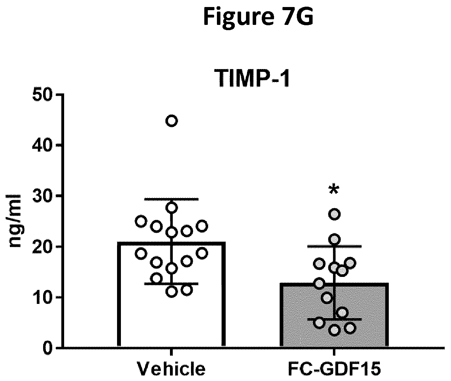

**Figure 7****.** ZSF1 obese rats treated with FC-GDF15 for 12 weeks exhibit statistically significant decrease in systemic levels of CVD-related biomarkers vWF (**Figure 7A**), Myl3 (**Figure 7B**), osteopontin (**Figure 7C**), E-Selectin (**Figure 7D**), sICAM (**Figure 7E**), VEGF (**Figure 7F**) and TIMP-1 (**Figure 7G**) at the end of the study. Significance (* p <0.05) determined using an unpaired two-tailed t-test. N = 11 for Vehicle, N = 8 for GDF15-Fc treated groups, respectively.

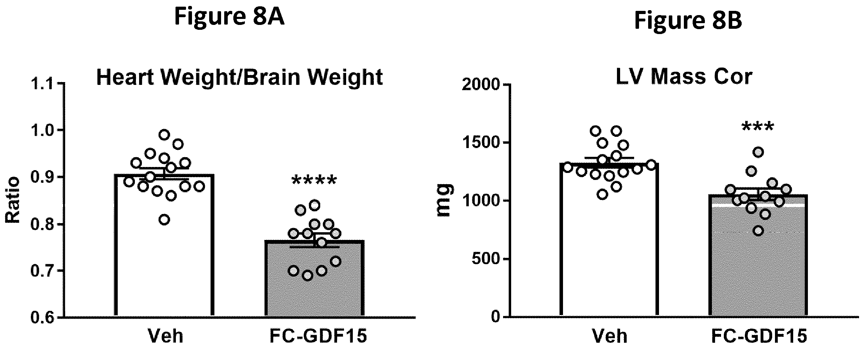

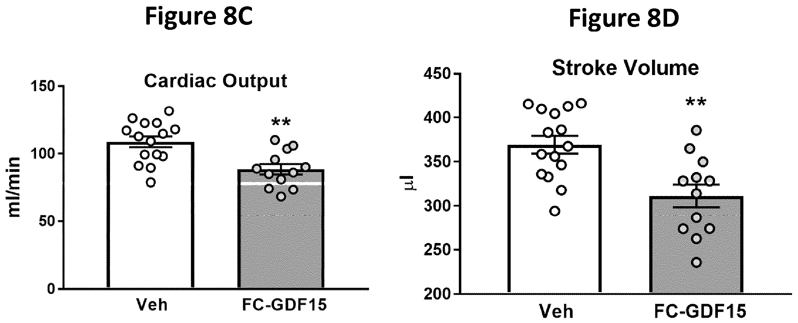

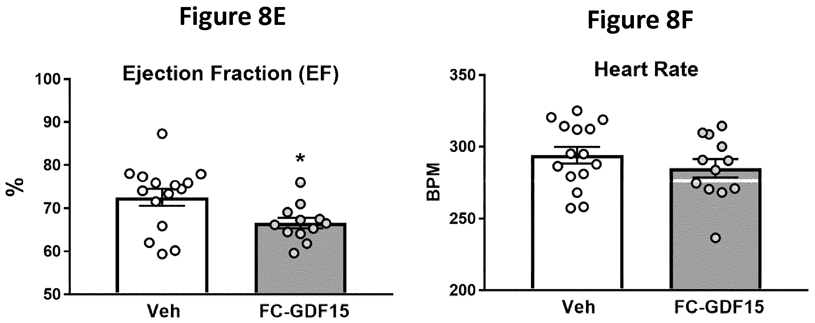

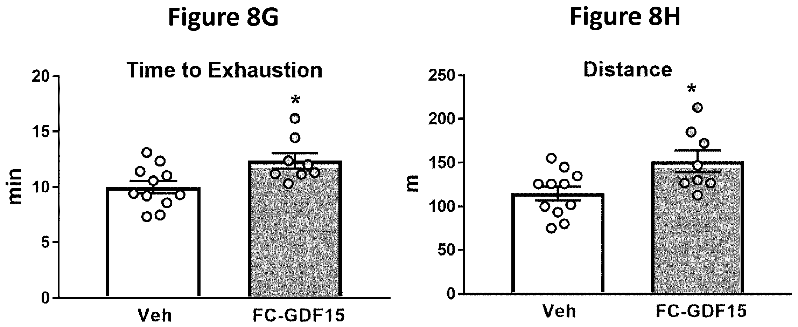

**Figure 8****.** Twelve weeks of FC-GDF15 treatment resulted in statistically significant decrease in heart weight and improved exercise capacity during treadmill challenge by the increase in running distance and the length of time to exhaustion. FC-GDF15 treatment resulted in significant decrease in heart to brain weight ratio (**Figure 8A**), LV mass (**Figure 8B**), cardiac output (**Figure 8C**), stroke volume (**Figure 8D**) and % ejection fraction echocardiographic parameters (**Figure 8E**), when compared to ZSF1 obese rats treated with Vehicle (**Fig. 7 A-E**). GDF15 treatment had no effect on heart rate (**Figure 8F**). After the twelve weeks of treatment, when exposed to a treadmill challenge, the FC-hGDF15 group demonstrated improved exercise capacity by exhibiting significantly longer running time (**Figure 8G**) and running distance (**Figure 8H**) vs. Vehicle group. Stars (*) indicate significance (* p <0.05, ** p <0.01, *** p <0.001, **** p <0.0001), unpaired two-tailed t-test).

### DETAILED DESCRIPTION

Provided herein is a GDF15 molecule for use in a method of treating a heart condition in a subject, the method comprising administering the GDF15 molecule to the subject, wherein the heart condition is heart failure with preserved ejection fraction (HFpEF).

Also provided is a GDF15 molecule for use in a method of treating a subject with cardiometabolic syndrome, the method comprising administering the GDF15 molecule to the subject. In some embodiments, the subject is obese, has impaired metabolism, pancreatic dysfunction, hypertension, diastolic dysfunction, HFpEF, decreased exercise capacity, or renal dysfunction.

In some embodiments, after treatment with the GDF15 molecule, the subject has a decreased heart weight, improved exercise capacity, or change in the level of adiponectin, sE-selectin, sICAM, Myl3, osteopontin, TIMP1, VEGF, or vWF, after treatment with the GDF15 molecule. In some embodiments, the level of adiponectin is increased. In some embodiments, the level of se-selectin, sICAM, Myl3, osteopontin, TIMP1, VEGF, vWF, or any combination thereof is decreased. In yet other embodiments, the subject has decreased heart to brain weight ratio, left ventricle (LV) mass, cardiac output, stroke volume, or % ejection fraction echocardiographic, after treatment with the GDF15 molecule.

In some embodiments, the GDF15 molecule is a GDF15-Fc fusion protein. The fusion protein can comprise a GDF15 region joined to an Fc region. In some embodiments, the GDF15 region is joined to the Fc via a linker. In some embodiments, the GDF15 region comprises wild type GDF15. Both the human and murine GDF15 have a signal peptide and prodomain. The nucleotide sequence for full length human GDF15 is:

atgcccgggc aagaactcag gacggtgaat ggctctcaga tgctcctggt gttgctggtg ctctcgtggc tgccgcatgg
gggcgccctg tctctggccg aggcgagccg cgcaagtttc ccgggaccct cagagttgca ctccgaagac tccagattcc
gagagttgcg gaaacgctac gaggacctgc taaccaggct gcgggccaac cagagctggg aagattcgaa caccgacctc
gtcccggccc ctgcagtccg gatactcacg ccagaagtgc ggctgggatc cggcggccac ctgcacctgc gtatctctcg
ggccgccctt cccgaggggc tccccgaggc ctcccgcctt caccgggctc tgttccggct gtccccgacg gcgtcaaggt
cgtgggacgt gacacgaccg ctgcggcgtc agctcagcct tgcaagaccc caggcgcccg cgctgcacct gcgactgtcg
ccgccgccgt cgcagtcgga ccaactgctg gcagaatctt cgtccgcacg gccccagctg gagttgcact tgcggccgca
agccgccagg gggcgccgca gagcgcgtgc gcgcaacggg gaccactgtc cgctcgggcc cgggcgttgc tgccgtctgc
acacggtccg cgcgtcgctg gaagacctgg gctgggccga ttgggtgctg tcgccacggg aggtgcaagt gaccatgtgc
atcggcgcgt gcccgagcca gttccgggcg gcaaacatgc acgcgcagat caagacgagc ctgcaccgcc tgaagcccga
cacggtgcca gcgccctgct gcgtgcccgc cagctacaat cccatggtgc tcattcaaaa gaccgacacc ggggtgtcgc
tccagaccta tgatgacttg ttagccaaag actgccactg catatga (SEQ ID NO: 1)

The amino acid sequence for full length human GDF15 (308 amino acids) is:

MPGQELRTVNGSQMLLVLLVLSWLPHGGALSLAEASRASFPGPSELHSEDSRFRELR
KRYEDLLTRLRANQSWEDSNTDLVPAPAVRILTPEVRLGSGGHLHLRISRAALPEGLP
EASRLHRALFRLSPTASRSWDVTRPLRRQLSLARPQAPALHLRLSPPPSQSDQLLAESS
SARPQLELHLRPQAARGRRRARARNGDHCPLGPGRCCRLHTVRASLEDLGWADWV
LSPREVQVTMCIGACPSQFRAANMHAQIKTSLHRLKPDTVPAPCCVPASYNPMVLIQ
KTDTGVSLQTYDDLLAKDCHCI (SEQ ID NO: 2)

The nucleotide sequence for human GDF15 without its signal sequence is:

ctgtctctgg ccgaggcgag ccgcgcaagt ttcccgggac cctcagagtt gcactccgaa gactccagat tccgagagtt
gcggaaacgc tacgaggacc tgctaaccag gctgcgggcc aaccagagct gggaagattc gaacaccgac ctcgtcccgg
cccctgcagt ccggatactc acgccagaag tgcggctggg atccggcggc cacctgcacc tgcgtatctc tcgggccgcc
cttcccgagg ggctccccga ggcctcccgc cttcaccggg ctctgttccg gctgtccccg acggcgtcaa ggtcgtggga
cgtgacacga ccgctgcggc gtcagctcag ccttgcaaga ccccaggcgc ccgcgctgca cctgcgactg
tcgccgccgc cgtcgcagtc ggaccaactg ctggcagaat cttcgtccgc acggccccag ctggagttgc acttgcggcc
gcaagccgcc agggggcgcc gcagagcgcg tgcgcgcaac ggggaccact gtccgctcgg gcccgggcgt
tgctgccgtc tgcacacggt ccgcgcgtcg ctggaagacc tgggctgggc cgattgggtg ctgtcgccac gggaggtgca
agtgaccatg tgcatcggcg cgtgcccgag ccagttccgg gcggcaaaca tgcacgcgca gatcaagacg agcctgcacc
gcctgaagcc cgacacggtg ccagcgccct gctgcgtgcc cgccagctac aatcccatgg tgctcattca aaagaccgac
accggggtgt cgctccagac ctatgatgac ttgttagcca aagactgcca ctgcatatga (SEQ ID NO: 3)

The amino acid sequence for human GDF15 without its 29 amino acid signal sequence (279 amino acids) is:

LSLAEASRASFPGPSELHSEDSRFRELRKRYEDLLTRLRANQSWEDSNTDLVPAPAVR
ILTPEVRLGSGGHLHLRISRAALPEGLPEASRLHRALFRLSPTASRSWDVTRPLRRQLS
LARPQAPALHLRLSPPPSQSDQLLAESSSARPQLELHLRPQAARGRRRARARNGDHCP
LGPGRCCRLHTVRASLEDLGWADWVLSPREVQVTMCIGACPSQFRAANMHAQIKTS
LHRLKPDTVPAPCCVPASYNPMVLIQKTDTGVSLQTYDDLLAKDCHCI (SEQ ID NO:
4)

The nucleotide sequence for human GDF15 without its signal peptide or prodomain is:

gcgcgcaacggggaccactgtccgctcgggcccgggcgttgctgccgtctgcacacggtccgcgcgtcgctggaagacctgggct
gggccgattgggtgctgtcgccacgggaggtgcaagtgaccatgtgcatcggcgcgtgcccgagccagttccgggcggcaaacatg
cacgcgcagatcaagacgagcctgcaccgcctgaagcccgacacggtgccagcgccctgctgcgtgcccgccagctacaatcccat
ggtgctcattcaaaagaccgacaccggggtgtcgctccagacctatgatgacttgttagccaaagactgccactgcatatga (SEQ
ID NO: 5)

The amino acid sequence for human GDF15 without its signal peptide or prodomain (the active domain of GDF15 of 112 amino acids) is:

ARNGDHCPLGPGRCCRLHTVRASLEDLGWADWVLSPREVQVTMCIGACPSQFRAAN
MHAQIKTSLHRLKPDTVPAPCCVPASYNPMVLIQKTDTGVSLQTYDDLLAKDCHCI
(SEQ ID NO: 6)

The nucleotide sequence for full length murine GDF15 is:

atggccccgc ccgcgctcca ggcccagcct ccaggcggct ctcaactgag gttcctgctg ttcctgctgc tgttgctgct
gctgctgtca tggccatcgc agggggacgc cctggcaatg cctgaacagc gaccctccgg ccctgagtcc caactcaacg
ccgacgagct acggggtcgc ttccaggacc tgctgagccg gctgcatgcc aaccagagcc gagaggactc
gaactcagaa ccaagtcctg acccagctgt ccggatactc agtccagagg tgagattggg gtcccacggc cagctgctac
tccgcgtcaa ccgggcgtcg ctgagtcagg gtctccccga agcctaccgc gtgcaccgag cgctgctcct gctgacgccg
acggcccgcc cctgggacat cactaggccc ctgaagcgtg cgctcagcct ccggggaccc cgtgctcccg cattacgcct
gcgcctgacg ccgcctccgg acctggctat gctgccctct ggcggcacgc agctggaact gcgcttacgg
gtagccgccg gcagggggcg ccgaagcgcg catgcgcacc caagagactc gtgcccactg ggtccggggc gctgctgtca
cttggagact gtgcaggcaa ctcttgaaga cttgggctgg agcgactggg tgctgtcccc gcgccagctg cagctgagca
tgtgcgtggg cgagtgtccc cacctgtatc gctccgcgaa cacgcatgcg cagatcaaag cacgcctgca tggcctgcag
cctgacaagg tgcctgcccc gtgctgtgtc ccctccagct acaccccggt ggttcttatg cacaggacag acagtggtgt
gtcactgcag acttatgatg acctggtggc ccggggctgc cactgcgctt ga (SEQ ID NO: 7)

The amino acid sequence for full length murine GDF15 (303 amino acids) is:

MAPPALQAQPPGGSQLRFLLFLLLLLLLLSWPSQGDALAMPEQRPSGPESQLNADEL
RGRFQDLLSRLHANQSREDSNSEPSPDPAVRILSPEVRLGSHGQLLLRVNRASLSQGL
PEAYRVHRALLLLTPTARPWDITRPLKRALSLRGPRAPALRLRLTPPPDLAMLPSGGT
QLELRLRVAAGRGRRSAHAHPRDSCPLGPGRCCHLETVQATLEDLGWSDWVLSPRQ
LQLSMCVGECPHLYRSANTHAQIKARLHGLQPDKVPAPCCVPSSYTPVVLMHRTDS
GVSLQTYDDLVARGCHCA (SEQ ID NO: 8)

The nucleotide sequence for murine GDF15 without its signal sequence is:

tcgcagggggacgccctggcaatgcctgaacagcgaccctccggccctgagtcccaactcaacgccgacgagctacggggtcgctt
ccaggacctgctgagccggctgcatgccaaccagagccgagaggactcgaactcagaaccaagtcctgacccagctgtccggatac
tcagtccagaggtgagattggggtcccacggccagctgctactccgcgtcaaccgggcgtcgctgagtcagggtctccccgaagcct
accgcgtgcaccgagcgctgctcctgctgacgccgacggcccgcccctgggacatcactaggcccctgaagcgtgcgctcagcctc
cggggaccccgtgctcccgcattacgcctgcgcctgacgccgcctccggacctggctatgctgccctctggcggcacgcagctgga
actgcgcttacgggtagccgccggcagggggcgccgaagcgcgcatgcgcacccaagagactcgtgcccactgggtccggggcg
ctgctgtcacttggagactgtgcaggcaactcttgaagacttgggctggagcgactgggtgctgtccccgcgccagctgcagctgagc
atgtgcgtgggcgagtgtccccacctgtatcgctccgcgaacacgcatgcgcagatcaaagcacgcctgcatggcctgcagcctgac

aaggtgcctgccccgtgctgtgtcccctccagctacaccccggtggttcttatgcacaggacagacagtggtgtgtcactgcagacttat
gatgacctggtggcccggggctgccactgcgcttga (SEQ ID NO: 9)

The amino acid sequence for murine GDF15 without its 32 amino acid signal sequence (271 amino acids) is:

SQGDALAMPEQRPSGPESQLNADELRGRFQDLLSRLHANQSREDSNSEPSPDPAVRIL
SPEVRLGSHGQLLLRVNRASLSQGLPEAYRVHRALLLLTPTARPWDITRPLKRALSLR
GPRAPALRLRLTPPPDLAMLPSGGTQLELRLRVAAGRGRRSAHAHPRDSCPLGPGRC
CHLETVQATLEDLGWSDWVLSPRQLQLSMCVGECPHLYRSANTHAQIKARLHGLQP
DKVPAPCCVPSSYTPVVLMHRTDSGVSLQTYDDLVARGCHCA (SEQ ID NO: 10)

The nucleotide sequence for murine GDF15 without its signal sequence or prodomain is:

agcgcgcatgcgcacccaagagactcgtgcccactgggtccggggcgctgctgtcacttggagactgtgcaggcaactcttgaagac
ttgggctggagcgactgggtgctgtccccgcgccagctgcagctgagcatgtgcgtgggcgagtgtccccacctgtatcgctccgcg
aacacgcatgcgcagatcaaagcacgcctgcatggcctgcagcctgacaaggtgcctgccccgtgctgtgtcccctccagctacacc
ccggtggttcttatgcacaggacagacagtggtgtgtcactgcagacttatgatgacctggtggcccggggctgccactgcgcttga
(SEQ ID NO: 11)

The amino acid sequence for murine GDF15 without its signal peptide or prodomain (active domain of 115 amino acids) is:

SAHAHPRDSCPLGPGRCCHLETVQATLEDLGWSDWVLSPRQLQLSMCVGECPHLYR
SANTHAQIKARLHGLQPDKVPAPCCVPSSYTPVVLMHRTDSGVSLQTYDDLVARGC
HCA (SEQ ID NO: 12)

In some embodiments, the GDF15 molecule comprises a GDF15 region comprising an active domain of GDF15, *e.g.,* GDF15 without its signal peptide or prodomain. In some embodiments, the GDF15 region comprises the amino acid sequence of SEQ ID NO: 6 or 12. In some embodiments, the GDF15 region comprises a GDF15 sequence with one or more mutations, such as at least one mutation in the active domain of GDF15. In particular embodiments, the mutation or mutations do not reduce or eliminate the activity of GDF15. In some embodiments, the GDF15 region comprises a mutation in the active domain of human GDF15. In one embodiment, the mutation is a deletion of the first three amino acids of the active domain, such as "GDF15(Δ3)" which is an active domain of human GDF15 in which the first three amino acids removed (*i.e.,* SEQ ID NO: 13).

In some embodiments, the GDF15 region comprises a mutation of the asparagine at position 3 (N3) of the active domain of human GDF15 (SEQ ID NO: 6). An N3 mutation can refer to the mutation of the asparagine residue at position 3 of SEQ ID NO: 6 or the mutation of an asparagine residue corresponding to the asparagine at position 3 of SEQ ID NO: 6 in a GDF15 amino acid sequence. In some embodiments, the asparagine at position 3 is mutated to glutamine (N3Q) or aspartate (N3D). Accordingly, in some embodiments, the GDF15 molecule comprises a GDF15 region of GDF15(N3Q), which has the amino acid sequence of SEQ ID NO: 14. In other embodiments, the GDF15 molecule comprises a GDF15 region of GDF15(N3D), which has the amino acid sequence of SEQ ID NO: 15. In some embodiments, the GDF15 region comprises a mutation of the aspartate at position 5 (D5) of the active domain of human GDF15 (SEQ ID NO: 6). A D5 mutation can refer to the mutation of the aspartate residue at position 5 of SEQ ID NO: 6 or the mutation of an aspartate residue corresponding to the aspartate at position 5 of SEQ ID NO: 6 in a GDF15 amino acid sequence. In one embodiment, the aspartate at position 5 is mutated to glutamate (D5E). Accordingly, in some embodiments, the GDF15 molecule comprises a GDF15 region of GDF15(D5E), which has the amino acid sequence of SEQ ID NO: 16.

In yet other embodiments, the GDF15 region comprises a combination of mutations, such as a combination of Δ3 and D5 mutations, *e.g.*, GDF15(Δ3/D5E) (SEQ ID NO: 17) or a combination ofN3 and D5 mutations, *e.g*., GDF15(N3D/D5E) or GDF15(N3Q/D5E). In, the GDF15 region comprises the amino acid sequence of SEQ ID NO: 18.

Table 1 provides examples of GDF15 regions that can be used in the GDF15 molecules.

**Table 1: GDF15 Regions**

| **SEQ ID NO:** | **Designation** | **Sequence** |
|---|---|---|
| 6 | GDF15 | ARNGDHCPLGPGRCCRLHTVRASLEDLGWADWVLSPREVQ VTMCIGACPSQFRAANMHAQIKTSLHRLKPDTVPAPCCVPA SYNPMVLIQKTDTGVSLQTYDDLLAKDCHCI |
| 13 | GDF15(Δ3) | GDHCPLGPGRCCRLHTVRASLEDLGWADWVLSPREVQVT MCIGACPSQFRAANMHAQIKTSLHRLKPDTVPAPCCVPASY NPMVLIQKTDTGVSLQTYDDLLAKDCHCI |
| 14 | GDF15(N3Q) | ARQGDHCPLGPGRCCRLHTVRASLEDLGWADWVLSPREVQ VTMCIGACPSQFRAANMHAQIKTSLHRLKPDTVPAPCCVPA SYNPMVLIQKTDTGVSLQTYDDLLAKDCHCI |
| 15 | GDF15(N3D) | ARDGDHCPLGPGRCCRLHTVRASLEDLGWADWVLSPREVQ VTMCIGACPSQFRAANMHAQIKTSLHRLKPDTVPAPCCVPA SYNPMVLIQKTDTGVSLQTYDDLLAKDCHCI |
| 16 | GDF15(D5E) | ARNGEHCPLGPGRCCRLHTVRASLEDLGWADWVLSPREVQ VTMCIGACPSQFRAANMHAQIKTSLHRLKPDTVPAPCCVPA SYNPMVLIQKTDTGVSLQTYDDLLAKDCHCI |
| 17 | GDF15(Δ3/D5E) | GEHCPLGPGRCCRLHTVRASLEDLGWADWVLSPREVQVTM CIGACPSQFRAANMHAQIKTSLHRLKPDTVPAPCCVPASYN PMVLIQKTDTGVSLQTYDDLLAKDCHCI |
| 18 | GDF15(N3Q/D5E) | ARQGEHCPLGPGRCCRLHTVRASLEDLGWADWVLSPREVQ VTMCIGACPSQFRAANMHAQIKTSLHRLKPDTVPAPCCVPA SYNPMVLIQKTDTGVSLQTYDDLLAKDCHCI |

In some embodiments, the GDF15 molecule is fused to an Fc directly. In other embodiments, the Fc is fused to the GDF15 molecule via a linker. In some embodiments, the linker is a G4S (SEQ ID NO: 19) linker. In other embodiments, the linker is a G4Q (SEQ ID NO: 24) linker. The linker can be a (G4S)n or (G4Q)n linker, wherein n is greater than 0. In some embodiments, the fusion protein has a linker that is a G4A (SEQ ID NO: 58) linker, such as a (G4A)n linker, wherein n is greater than 0. In some embodiments, n is 1 or 2. In some embodiments, n is greater than 2, such as 3, 4, 5, 6, 7, or 8. In some embodiments, the linker comprises the amino acid sequence of SEQ ID NO: 19, 20, 21, 22, 23, 24, 25 or 58, as shown in Table 2.

**Table 2: Linkers**

| **SEQ ID NO:** | **Designation** | **Sequence** |
|---|---|---|
| 19 | G4S | GGGGS |
| 20 | (G4S)2 | GGGGSGGGGS |
| 21 | (G4S)4 | GGGGSGGGGSGGGGSGGGGS |
| 22 | (G4S)8 | GGGGSGGGGSGGGGSGGGGSGGGGSGGGGSGGGGSGGGGS |
| 23 | G4 | GGGG |
| 24 | G4Q | GGGGQ |
| 25 | (G4Q)4 | GGGGQGGGGQGGGGQGGGGQ |
| 58 | G4A | GGGGA |

In some embodiments, the GDF15 molecule comprises an Fc region. The Fc region can comprise or be derived from the Fc domain of a heavy chain of an antibody. In some embodiments, the Fc region may comprise an Fc domain with a mutation, such as a charged pair mutation, a mutation in a glycosylation site or the inclusion of an unnatural amino acid. The Fc region can be derived from a human IgG constant domain of IgG1, IgG2, IgG3 or IgG4. In some embodiments, the Fc region comprises the constant domain of an IgA, IgD, IgE, and IgM heavy chain.

In some embodiments, the Fc region comprises an Fc domain with a charged pair mutation. By introducing a mutation resulting in a charged Fc region, the GDF15 molecule can dimerize with a corresponding Fc molecule having the opposite charge. For example, an aspartate-to-lysine mutation (E356K, wherein 356 is the position using EU numbering, and corresponds to the positions as noted in Tables 3-5) and a glutamate-to-lysine mutation (D399K wherein 399 is the position using EU numbering, and corresponds to positions as noted in Tables 3-5) can be introduced into the Fc region that is joined to a GDF15 region, optionally via a linker, resulting in a positively charged Fc region for the GDF15 molecule. Lysine-to-aspartate mutations (K392D, K409D; wherein 392 and 409 are the positions using EU numbering and corresponds to the positions as noted in Tables 3-5) can be introduced into an Fc domain of a separate molecule, resulting in a negatively charged Fc molecule. The aspartate residues in the negatively charged Fc molecule can associate with the lysine residues of the positively charged Fc region of the GDF15 molecule through electrostatic force, facilitating formation of Fc heterodimers between the Fc region of the GDF15 molecule and the Fc molecule, while reducing or preventing formation of Fc homodimers between the Fc regions of the GDF15 molecules or between Fc molecules.

In some embodiments, one or more lysine-to-aspartate mutations (K392D, K409D) are introduced into the Fc region that is joined to a GDF15 region, optionally via a linker and an aspartate-to-lysine mutation (E356K) and a glutamate-to-lysine mutation (D399K) is introduced into the Fc domain of another molecule. The aspartate residues in the Fc region of the GDF15 molecule can associate with the lysine residues of the Fc molecule through electrostatic force, facilitating formation of Fc heterodimers between the Fc region of the GDF15 molecule and the Fc molecule, and reducing or preventing formation of Fc homodimers between the Fc regions of the GDF15 molecules or between Fc molecules.

In some embodiments, the GDF15 molecule comprises an Fc region comprising an Fc domain with a mutated hinge region. In some embodiments, the Fc domain comprises a deletion in the hinge. In some embodiments, ten amino acids from the hinge are deleted, e.g., FcΔ10. In other embodiments, sixteen amino acids from the hinge are deleted, e.g., FcΔ16. In some embodiments, the Fc domain comprises a hinge deletion (e.g., FcΔ10 or FcΔ16) and a charged pair mutation, such that the Fc domain is positively or negatively charged. For example, the Fc domain can comprise a ten-amino acid deletion in the hinge and lysine-to-aspartate mutations (K392D, K409D), such as FcΔ10(-). In another embodiment, the Fc domain can comprise a ten-amino acid deletion in the hinge and an aspartate-to-lysine mutation (E356K) and a glutamate-to-lysine mutation (D399K), such as an FcΔ10(+). In another embodiment, the Fc domain can comprise a sixteen-amino acid deletion in the hinge and lysine-to-aspartate mutations (K392D, K409D), such as FcΔ16(-). In another embodiment, the Fc domain can comprise a sixteen- amino acid deletion in the hinge and an aspartate-to-lysine mutation (E356K) and a glutamate-to-lysine mutation (D399K), such as an FcΔ16(+).

In some embodiments, an Fc molecule comprising a hinge deletion and a charged pair mutation heterodimerizes with such a GDF15 molecule. For example, the Fc molecule can have a hinge deletion and charged pair mutation that complements the hinge deletion and charged pair mutation of the Fc region of a GDF15 molecule. For example, an Fc molecule can comprise an Fc domain with a ten-amino acid deletion in the hinge and lysine-to-aspartate mutations (K392D, K409D), such as FcΔ10(-), which can optionally comprise a C-terminal lysine (e.g., FcΔ10(-, K)). The Fc molecule can heterodimerize with a GDF15 molecule that comprises an FcΔ10(+). In another embodiment, the Fc molecule can comprise a ten-amino acid deletion in the hinge and an aspartate-to-lysine mutation (E356K) and a glutamate-to-lysine mutation (D399K), such as an FcΔ10(+), which can optionally comprise a C-terminal lysine (e.g., FcΔ10(+, K)). The Fc molecule can heterodimerize with a GDF15 molecule that comprises an FcΔ10(-). In another embodiment, the Fc molecule can comprise a sixteen-amino acid deletion in the hinge and lysine-to-aspartate mutations (K392D, K409D), such as FcΔ16(-), which can optionally comprise a C-terminal lysine (e.g., FcΔ16(-, K)). The Fc molecule which can heterodimerize with a GDF15 molecule that comprises an FcΔ16(+). In another embodiment, the Fc molecule can comprise a sixteen-amino acid deletion in the hinge and an aspartate-to-lysine mutation (E356K) and a glutamate-to-lysine mutation (D399K), such as an FcΔ16(+), which can optionally comprise a C-terminal lysine (e.g., FcΔ16(-, K)). The Fc molecule can heterodimerize with a GDF15 molecule that comprises an FcΔ16(-).

In some embodiments, the Fc region or Fc molecule comprises an Fc domain with an L234A and/or L235A mutation, wherein 234 and 235 are the positions using EU numbering and corresponds to the positions as noted in Tables 3-5. The Fc domain can comprise an L234A mutation, an L235A mutation, a charged pair mutation, a hinge deletion, or any combination thereof. In some embodiments, the Fc domain comprises both an L234A mutation and an L235A mutation. In some embodiments, the Fc domain comprises a hinge deletion, an L234A mutation, an L235A mutation, and a charged pair mutation, such as FcΔ10(+, L234A/L235A), FcΔ10(-, L234A/L235A), FcΔ16(+, L234A/L235A), or FcΔ16(-, L234A/L235A). In some embodiments, the Fc domain comprises an optional C-terminal lysine, e.g., FcΔ10(+,K,L234A/L235A), FcΔ10(-,K,L234A/L235A), FcΔ16(+,K,L234A/L235A), or FcΔ16(-,K,L234A/L235A).

In some embodiments, the Fc region or Fc molecule comprises an Fc domain with a "cysteine clamp." A cysteine clamp mutation involves the introduction of a cysteine into the Fc domain at a specific location through mutation so that when incubated with another Fc domain that also has a cysteine introduced at a specific location through mutation, a disulfide bond (cysteine clamp) may be formed between the two Fc domains (e.g., between an FcΔ16 (+) domain having a "cysteine clamp" mutation and an FcΔ16(-) domain having a "cysteine clamp" mutation). The cysteine can be introduced into the CH3 domain of an Fc domain. In some embodiments, an Fc domain may contain one or more such cysteine clamp mutations. In one embodiment, a cysteine clamp is provided by introducing a serine to cysteine mutation (S354C, wherein 354 is the position using EU numbering, and corresponds to the position as noted in Tables 3-5) into a first Fc domain and a tyrosine to cysteine mutation (Y349C, wherein 349 is the position using EU numbering, and corresponds to the position as noted in Tables 3-5) into a second Fc domain. In one embodiment, a GDF15 molecule comprises an Fc region comprising an Fc domain with a cysteine clamp, a negatively charged pair mutation and a sixteen-amino acid hinge deletion (e.g., GDF15- FcΔ16(-,CC)), and an Fc molecule comprising an Fc domain comprising a cysteine clamp, a positively charged pair mutation and a sixteen-amino acid hinge deletion, and an optional C-terminal lysine (e.g., FcΔ16(+,K,CC)). The cysteine clamp may augment the heterodimerization of the GDF-Fc molecule with the Fc molecule.

Examples of Fc regions that can be used in a GDF15 molecule are shown in Table 3.

**Table 3: Fc Regions**

| **SEQ ID NO:** | **Designation** | **Sequence** |
|---|---|---|
| 26 | FcΔ10(-) | APELLGGPSVFLFPPKPKDTLMISRTPEVTCVVVDVSHE DPEVKFNWYVDGVEVHNAKTKPREEQYNSTYRVVSVL TVLHQDWLNGKEYKCKVSNKALPAPIEKTISKAKGQPR EPQVYTLPPSREEMTKNQVSLTCLVKGFYPSDIAVEWES NGQPENNYDTTPPVLDSDGSFFLYSDLTVDKSRWQQGN VFSCSVMHEALHNHYTQKSLSLSPG *Underlined and bolded residues are K392D and K409D mutations.* |
| 27 | FcΔ10(+) | APELLGGPSVFLFPPKPKDTLMISRTPEVTCVVVDVSHE DPEVKFNWYVDGVEVHNAKTKPREEQYNSTYRVVSVL TVLHQDWLNGKEYKCKVSNKALPAPIEKTISKAKGQPR EPQVYTLPPSRKEMTKNQVSLTCLVKGFYPSDIAVEWES NGQPENNYKTTPPVLKSDGSFFLYSKLTVDKSRWQQGN VFSCSVMHEALHNHYTQKSLSLSPG *Underlined and bolded residues are E356K and D399K mutations.* |
| 28 | FcΔ10(-,CC) | APELLGGPSVFLFPPKPKDTLMISRTPEVTCVVVDVSHE DPEVKFNWYVDGVEVHNAKTKPREEQYNSTYRVVSVL TVLHQDWLNGKEYKCKVSNKALPAPIEKTISKAKGQPR EPQVCTLPPSREEMTKNQVSLTCLVKGFYPSDIAVEWES NGQPENNYDTTPPVLDSDGSFFLYSDLTVDKSRWQQGN VFSCSVMHEALHNHYTQKSLSLSPG *Underlined and italicized residue is Y349C mutation; underlined and bolded residues are K392D and K409D mutations.* |
| 29 | FcΔ16(-,CC) | GPSVFLFPPKPKDTLMISRTPEVTCVVVDVSHEDPEVKF NWYVDGVEVHNAKTKPREEQYNSTYRVVSVLTVLHQD WLNGKEYKCKVSNKALPAPIEKTISKAKGQPREPQVCTL PPSREEMTKNQVSLTCLVKGFYPSDIAVEWESNGQPENN YDTTPPVLDSDGSFFLYSDLTVDKSRWQQGNVFSCSVM HEALHNHYTQKSLSLSPG *Underlined and italicized residue is Y349C mutation; underlined and bolded residues are K392D and K409D mutations.* |
| 30 | FcΔ16(-) | GPSVFLFPPKPKDTLMISRTPEVTCVVVDVSHEDPEVKF NWYVDGVEVHNAKTKPREEQYNSTYRVVSVLTVLHQD WLNGKEYKCKVSNKALPAPIEKTISKAKGQPREPQVYT LPPSREEMTKNQVSLTCLVKGFYPSDIAVEWESNGQPEN NYDTTPPVLDSDGSFFLYSDLTVDKSRWQQGNVFSCSV MHEALHNHYTQKSLSLSPG *Underlined and bolded residues are K392D and K409D mutations.* |
| 31 | FcΔ10(-,L234A/L235A) | APEAAGGPSVFLFPPKPKDTLMISRTPEVTCVVVDVSHE DPEVKFNWYVDGVEVHNAKTKPREEQYNSTYRVVSVL TVLHQDWLNGKEYKCKVSNKALPAPIEKTISKAKGQPR EPQVYTLPPSREEMTKNQVSLTCLVKGFYPSDIAVEWES NGQPENNYDTTPPVLDSDGSFFLYSDLTVDKSRWQQGN VFSCSVMHEALHNHYTQKSLSLSPG *Underlined and italicized residues are L234A and L235A mutations; underlined and bolded residues are K392D and K409D mutations.* |

Examples of Fc molecules are shown in Table 4, in which the C-terminal lysine is optional.

**Table 4: Fc Molecules**

| **SEQ ID NO:** | **Designation** | **Sequence** |
|---|---|---|
| 32 | FcΔ10(+,K) | APELLGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAKGQPREPQVYTL PPSRKEMTKNQVSLTCLVKGFYPSDIAVEWESNGQPENNY KTTPPVLKSDGSFFLYSKLTVDKSRWQQGNVFSCSVMHEA LHNHYTQKSLSLSPGK *Underlined and bolded residues are E356K and D399K mutations.* |
| 33 | FcΔ10(-,K) | APELLGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAKGQPREPQVYTL PPSREEMTKNQVSLTCLVKGFYPSDIAVEWESNGQPENNY DTTPPVLDSDGSFFLYSDLTVDKSRWQQGNVFSCSVMHEA LHNHYTQKSLSLSPGK *Underlined and bolded residues are K392D and K409D mutations.* |
| 34 | FcΔ10(+,K,C C) | APELLGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAKGQPREPQVYTL PPCRKEMTKNQVSLTCLVKGFYPSDIAVEWESNGQPENNY KTTPPVLKSDGSFFLYSKLTVDKSRWQQGNVFSCSVMHEA LHNHYTQKSLSLSPGK *Underlined and italicized residue is S354C mutation; underlined and bolded residues are E356K and D399K mutations.* |
| 35 | FcΔ16(+,K,C C) | GPSVFLFPPKPKDTLMISRTPEVTCVVVDVSHEDPEVKFNW YVDGVEVHNAKTKPREEQYNSTYRVVSVLTVLHQDWLNG KEYKCKVSNKALPAPIEKTISKAKGQPREPQVYTLPPCRKE MTKNQVSLTCLVKGFYPSDIAVEWESNGQPENNYKTTPPV LKSDGSFFLYSKLTVDKSRWQQGNVFSCSVMHEALHNHY TQKSLSLSPGK *Underlined and italicized residue is S354C mutation; underlined and bolded residues are E356K and D399K mutations.* |
| 36 | FcΔ16(+,K) | GPSVFLFPPKPKDTLMISRTPEVTCVVVDVSHEDPEVKFNW YVDGVEVHNAKTKPREEQYNSTYRVVSVLTVLHQDWLNG KEYKCKVSNKALPAPIEKTISKAKGQPREPQVYTLPPSRKE MTKNQVSLTCLVKGFYPSDIAVEWESNGQPENNYKTTPPV LKSDGSFFLYSKLTVDKSRWQQGNVFSCSVMHEALHNHY TQKSLSLSPGK *Underlined and bolded residues are E356K and D399K mutations.* |
| 37 | FcΔ10(+,K,L2 34A/L235A) | APEAAGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAKGQPREPQVYTL PPSRKEMTKNQVSLTCLVKGFYPSDIAVEWESNGQPENNY KTTPPVLKSDGSFFLYSKLTVDKSRWQQGNVFSCSVMHEA LHNHYTQKSLSLSPGK *Underlined and italicized residues are L234A and L235A mutations; underlined and bolded residues are E356K and D399K mutations.* |

The Fc molecules can be used to dimerize with a molecule comprising a complementary Fc domain. For example, an Fc molecule of FcΔ10(+,K) can dimerize with a molecule comprising an Fc region comprising a ten-amino acid hinge deletion and a negatively charged pair mutation such as FcΔ10(-) (e.g., a GDF15 molecule comprising an Fc region of FcΔ10(-)). An Fc molecule of FcΔ10(-,K)can dimerize with a molecule comprising an Fc region comprising a ten-amino acid hinge deletion and a negatively charged pair mutation such as FcΔ10(+) (e.g., a GDF15 molecule comprising an Fc region of FcΔ10(+)).

An Fc molecule of FcΔ10(+,K,CC) can dimerize with a molecule comprising an Fc region comprising a ten-amino acid hinge deletion and a negatively charged pair mutation such as FcΔ10(-,CC) (e.g., a GDF15 molecule comprising an Fc region of FcΔ10(-, CC)). An Fc molecule of FcΔ16(+,K,CC) can dimerize with a molecule comprising an Fc region comprising a ten-amino acid hinge deletion and a negatively charged pair mutation such as FcΔ16(-, CC) (e.g., a GDF15 molecule comprising an Fc region of FcΔ16(-, CC)). An Fc molecule of FcΔ16(+,K) can dimerize with a molecule comprising an Fc region comprising a ten-amino acid hinge deletion and a negatively charged pair mutation such as FcΔ16(-) (e.g., a GDF15 molecule comprising an Fc region of FcΔ16(+)). An Fc molecule of FcΔ10(+,K,L234A/L235A) can dimerize with a molecule comprising an Fc region comprising a ten-amino acid hinge deletion and a negatively charged pair mutation such as FcΔ10(-,L234A/L235A) (e.g., a GDF15 molecule comprising an Fc region of FcΔ10(-, L234A/L235A)).

Examples of GDF15 molecules that are GDF15-Fc fusion proteins are shown in Table 5.

**Table 5: GDF15 Molecules**

| **GDF15-Fc Fusion Protein** | | | **GDF15-Fc Fusion Protein Components SEQ ID NOs** | | |
|---|---|---|---|---|---|
| **SEQ ID NO.** | **Designation** | **Sequence** | **Fc Region** | **Linker** | **GDF15 Region** |
| 38 | scFc-GDF15 | GGGERKSSVECPPCPAPPVA GPSVFLFPPKPKDTLMISRT PEVTCVVVDVSHEDPEVQF NWYVDGVEVHNAKTKPRE EQFNSTFRVVSVLTVVHQD WLNGKEYKCKVSNKGLPA PIEKTISKTKGQPREPQVYT LPPSREEMTKNQVSLTCLV KGFYPSDIAVEWESNGQPE NNYKTTPPMLDSDGSFFLY SKLTVDKSRWQQGNVFSCS VMHEALHNHYTQKSLSLSP GGGGGSGGGGSGGGGSGG GGSGGGGSGGGGSGGGGS GGGGSERKSSVECPPCPAPP VAGPSVFLFPPKPKDTLMIS RTPEVTCVVVDVSHEDPEV QFNWYVDGVEVHNAKTKP REEQFNSTFRVVSVLTVVH QDWLNGKEYKCKVSNKGL PAPIEKTISKTKGQPREPQV YTLPPSREEMTKNQVSLTC LVKGFYPSDIAVEWESNGQ PENNYKTTPPMLDSDGSFFL YSKLTVDKSRWQQGNVFS CSVMHEALHNHYTQKSLSL SPGSGGGGSGGGGSGGGGS GGGGSARNGDHCPLGPGRC CRLHTVRASLEDLGWADW VLSPREVQVTMCIGACPSQF RAANMHAQIKTSLHRLKPD TVPAPCCVPASYNPMVLIQ KTDTGVSLQTYDDLLAKDC HCI | --- | --- | --- |
| 39 | FcΔ10(-)-(G4S)4-GDF15 | APELLGGPSVFLFPPKPKDT LMISRTPEVTCVVVDVSHE DPEVKFNWYVDGVEVHNA KTKPREEQYNSTYRVVSVL TVLHQDWLNGKEYKCKVS NKALPAPIEKTISKAKGQPR EPQVYTLPPSREEMTKNQV SLTCLVKGFYPSDIAVEWES NGQPENNYDTTPPVLDSDG SFFLYSDLTVDKSRWQQGN VFSCSVMHEALHNHYTQKS LSLSPGGGGGSGGGGSGGG GSGGGGSARNGDHCPLGPG RCCRLHTVRASLEDLGWA DWVLSPREVQVTMCIGACP SQFRAANMHAQIKTSLHRL KPDTVPAPCCVPASYNPMV LIQKTDTGVSLQTYDDLLA KDCHCI *Underlined and bolded residues are K392D and K409D mutations.* | 26 | 21 | 6 |
| 40 | FcΔ10(+)-(G4)-GDF15 | APELLGGPSVFLFPPKPKDT LMISRTPEVTCVVVDVSHE DPEVKFNWYVDGVEVHNA KTKPREEQYNSTYRVVSVL TVLHQDWLNGKEYKCKVS NKALPAPIEKTISKAKGQPR EPQVYTLPPSRKEMTKNQV SLTCLVKGFYPSDIAVEWES NGQPENNYKTTPPVLKSDG SFFLYSKLTVDKSRWQQGN VFSCSVMHEALHNHYTQKS LSLSPGGGGGARNGDHCPL GPGRCCRLHTVRASLEDLG WADWVLSPREVQVTMCIG ACPSQFRAANMHAQIKTSL HRLKPDTVPAPCCVPASYN PMVLIQKTDTGVSLQTYDD LLAKDCHCI *Underlined and and bolded residues are E356K and D399K mutations.* | 27 | 23 | 6 |
| 41 | FcΔ10(-)-GDF15(Δ3) | APELLGGPSVFLFPPKPKDT LMISRTPEVTCVVVDVSHE DPEVKFNWYVDGVEVHNA KTKPREEQYNSTYRVVSVL TVLHQDWLNGKEYKCKVS NKALPAPIEKTISKAKGQPR EPQVYTLPPSREEMTKNQV SLTCLVKGFYPSDIAVEWES NGQPENNYDTTPPVLDSDG SFFLYSDLTVDKSRWQQGN VFSCSVMHEALHNHYTQKS LSLSPGGDHCPLGPGRCCRL HTVRASLEDLGWADWVLS PREVQVTMCIGACPSQFRA ANMHAQIKTSLHRLKPDTV PAPCCVPASYNPMVLIQKT DTGVSLQTYDDLLAKDCHC I *Underlined and bolded residues are K392D and K409D mutations.* | 26 | --- | 13 |
| 42 | FcΔ10(-)-GDF15(N3 D) | APELLGGPSVFLFPPKPKDT LMISRTPEVTCVVVDVSHE DPEVKFNWYVDGVEVHNA KTKPREEQYNSTYRVVSVL TVLHQDWLNGKEYKCKVS NKALPAPIEKTISKAKGQPR EPQVYTLPPSREEMTKNQV SLTCLVKGFYPSDIAVEWES NGQPENNYDTTPPVLDSDG SFFLYSDLTVDKSRWQQGN VFSCSVMHEALHNHYTQKS LSLSPGARDGDHCPLGPGR CCRLHTVRASLEDLGWAD WVLSPREVQVTMCIGACPS QFRAANMHAQIKTSLHRLK PDTVPAPCCVPASYNPMVLI QKTDTGVSLQTYDDLLAKD CHCI *Underlined and bolded residues are K392D and K409D mutations.* | 26 | --- | 15 |
| 43 | FcΔ10(-,CC)-GDF15(Δ3) | APELLGGPSVFLFPPKPKDT LMISRTPEVTCVVVDVSHE DPEVKFNWYVDGVEVHNA KTKPREEQYNSTYRVVSVL TVLHQDWLNGKEYKCKVS NKALPAPIEKTISKAKGQPR EPQVCTLPPSREEMTKNQV SLTCLVKGFYPSDIAVEWES NGQPENNYDTTPPVLDSDG SFFLYSDLTVDKSRWQQGN VFSCSVMHEALHNHYTQKS LSLSPGGDHCPLGPGRCCRL HTVRASLEDLGWADWVLS PREVQVTMCIGACPSQFRA ANMHAQIKTSLHRLKPDTV PAPCCVPASYNPMVLIQKT DTGVSLQTYDDLLAKDCHC I *Underlined and italicized residue is Y349C mutation; underlined and bolded residues are K392D and K409D mutations.* | 28 | --- | 13 |
| 44 | FcΔ10(-,CC)-GDF15(N3 D) | APELLGGPSVFLFPPKPKDT LMISRTPEVTCVVVDVSHE DPEVKFNWYVDGVEVHNA KTKPREEQYNSTYRVVSVL TVLHQDWLNGKEYKCKVS NKALPAPIEKTISKAKGQPR EPQVCTLPPSREEMTKNQV SLTCLVKGFYPSDIAVEWES NGQPENNYDTTPPVLDSDG SFFLYSDLTVDKSRWQQGN VFSCSVMHEALHNHYTQKS LSLSPGARDGDHCPLGPGR CCRLHTVRASLEDLGWAD WVLSPREVQVTMCIGACPS QFRAANMHAQIKTSLHRLK PDTVPAPCCVPASYNPMVLI QKTDTGVSLQTYDDLLAKD CHCI *Underlined and italicized residue is Y349C mutation; underlined and bolded residues are K392D and K409D mutations.* | 28 | --- | 15 |
| 45 | FcΔ16(-,CC)-GDF15(Δ3/ D5E) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVCT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GGEHCPLGPGRCCRLHTVR ASLEDLGWADWVLSPREV QVTMCIGACPSQFRAANMH AQIKTSLHRLKPDTVPAPCC VPASYNPMVLIQKTDTGVS LQTYDDLLAKDCHCI [?] *Underlined and italicized residue is Y349C mutation; underlined and bolded residues are K392D and K409D mutations.* | 29 | --- | 17 |
| 46 | FcΔ16(-,CC)-GDF15(N3 Q/D5E) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVCT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GARQGEHCPLGPGRCCRLH TVRASLEDLGWADWVLSP REVQVTMCIGACPSQFRAA NMHAQIKTSLHRLKPDTVP APCCVPASYNPMVLIQKTD TGVSLQTYDDLLAKDCHCI *Underlined and italicized residue is Y349C mutation; underlined and bolded residues are K392D and K409D mutations.* | 29 | --- | 18 |
| 47 | FcΔ16(-)-GDF15(N3 Q/D5E) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVYT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GARQGEHCPLGPGRCCRLH TVRASLEDLGWADWVLSP REVQVTMCIGACPSQFRAA NMHAQIKTSLHRLKPDTVP APCCVPASYNPMVLIQKTD TGVSLQTYDDLLAKDCHCI *Underlined and bolded residues are K392D and K409D mutations.* | 30 | --- | 18 |
| 48 | FcΔ16(-)-(G4Q)4-GDF15 | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVYT LPPSREEMTKNQVSLTCLV *Underlined and bolded residues are K392D and K409D mutations.* | 30 | 25 | 6 |
| 49 | FcΔ16(-)-(G4Q)4-GDF15(N3 Q) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVYT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GGGGGQGGGQGGGGQG GGGQARQGDHCPLGPGRC CRLHTVRASLEDLGWADW VLSPREVQVTMCIGACPSQF RAANMHAQIKTSLHRLKPD TVPAPCCVPASYNPMVLIQ KTDTGVSLQTYDDLLAKDC HCI *Underlined and bolded residues are K392D and K409D mutations.* | 30 | 25 | 14 |
| 50 | FcΔ16(-)-(G4Q)4-GDF15(N3 Q/D5E) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVYT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GGGGGQGGGGQGGGGQG GGGQARQGEHCPLGPGRCC RLHTVRASLEDLGWADWV LSPREVQVTMCIGACPSQFR AANMHAQIKTSLHRLKPDT VPAPCCVPASYNPMVLIQK TDTGVSLQTYDDLLAKDCH CI *Underlined and bolded residues are K392D and K409D mutations.* | 30 | 25 | 18 |
| 51 | FcΔ16(-)-(G4S)2-GDF15(N3 Q) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVYT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GGGGGSGGGGSARQGDHC PLGPGRCCRLHTVRASLED LGWADWVLSPREVQVTMC IGACPSQFRAANMHAQIKT SLHRLKPDTVPAPCCVPAS YNPMVLIQKTDTGVSLQTY DDLLAKDCHCI *Underlined and bolded residues are K392D and K409D mutations.* | 30 | 20 | 14 |
| 52 | FcΔ16(-)-(G4S)2-GDF15(N3 Q/D5E) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVYT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GGGGGSGGGGSARQGEHC PLGPGRCCRLHTVRASLED LGWADWVLSPREVQVTMC IGACPSQFRAANMHAQIKT SLHRLKPDTVPAPCCVPAS YNPMVLIQKTDTGVSLQTY DDLLAKDCHCI *Underlined and bolded residues are K392D and K409D mutations.* | 30 | 20 | 18 |
| 53 | FcΔ16(-)-G4S-GDF15(N3 Q) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVYT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GGGGGSARQGDHCPLGPG RCCRLHTVRASLEDLGWA DWVLSPREVQVTMCIGACP SQFRAANMHAQIKTSLHRL KPDTVPAPCCVPASYNPMV LIQKTDTGVSLQTYDDLLA KDCHCI *Underlined and bolded residues are K392D and K409D mutations.* | 30 | 19 | 14 |
| 54 | FcΔ16(-)-G4S-GDF15(N3 Q/D5E) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVYT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GGGGGSARQGEHCPLGPGR CCRLHTVRASLEDLGWAD WVLSPREVQVTMCIGACPS QFRAANMHAQIKTSLHRLK PDTVPAPCCVPASYNPMVLI QKTDTGVSLQTYDDLLAKD CHCI *Underlined and bolded residues are K392D and K409D mutations.* | 30 | 19 | 18 |
| 55 | FcΔ16(-)-GDF15(N3 Q) | GPSVFLFPPKPKDTLMISRT PEVTCVVVDVSHEDPEVKF NWYVDGVEVHNAKTKPRE EQYNSTYRVVSVLTVLHQD WLNGKEYKCKVSNKALPA PIEKTISKAKGQPREPQVYT LPPSREEMTKNQVSLTCLV KGFYPSDIAVEWESNGQPE NNYDTTPPVLDSDGSFFLYS DLTVDKSRWQQGNVFSCS VMHEALHNHYTQKSLSLSP GARQGDHCPLGPGRCCRLH TVRASLEDLGWADWVLSP REVQVTMCIGACPSQFRAA NMHAQIKTSLHRLKPDTVP APCCVPASYNPMVLIQKTD TGVSLQTYDDLLAKDCHCI *Underlined and bolded residues are K392D and K409D mutations.* | 30 | --- | 14 |
| 56 | FcΔ10(-,L234A/L23 5A)-(G4Q)4-GDF15(N3 Q) | APEAAGGPSVFLFPPKPKDT LMISRTPEVTCVVVDVSHE DPEVKFNWYVDGVEVHNA KTKPREEQYNSTYRVVSVL TVLHQDWLNGKEYKCKVS NKALPAPIEKTISKAKGQPR EPQVYTLPPSREEMTKNQV SLTCLVKGFYPSDIAVEWES NGQPENNYDTTPPVLDSDG SFFLYSDLTVDKSRWQQGN VFSCSVMHEALHNHYTQKS LSLSPGGGGGQGGGGQGG GGQGGGGQARQGDHCPLG PGRCCRLHTVRASLEDLGW ADWVLSPREVQVTMCIGAC PSQFRAANMHAQIKTSLHR LKPDTVPAPCCVPASYNPM VLIQKTDTGVSLQTYDDLL AKDCHCI *Underlined and italicized residues are L234A and L235A mutations; underlined and bolded residues are K392D and K409D mutations.* | 31 | 25 | 14 |
| 57 | FcΔ10(-,L234A/L23 5A)-(G4Q)4-GDF15(N3 Q/D5E) | APEAAGGPSVFLFPPKPKDT LMISRTPEVTCVVVDVSHE DPEVKFNWYVDGVEVHNA KTKPREEQYNSTYRVVSVL TVLHQDWLNGKEYKCKVS NKALPAPIEKTISKAKGQPR EPQVYTLPPSREEMTKNQV SLTCLVKGFYPSDIAVEWES NGQPENNYDTTPPVLDSDG SFFLYSDLTVDKSRWQQGN VFSCSVMHEALHNHYTQKS LSLSPGGGGGQGGGGQGG GGQGGGGQARQGEHCPLG PGRCCRLHTVRASLEDLGW ADWVLSPREVQVTMCIGAC PSQFRAANMHAQIKTSLHR LKPDTVPAPCCVPASYNPM VLIQKTDTGVSLQTYDDLL AKDCHCI *Underlined and italicized residues are L234A and L235A mutations; underlined and bolded residues are K392D and K409D mutations.* | 31 | 25 | 18 |

In some embodiments, the fusion protein is an scFc-GDF15 in which the GDF15 region is joined to two Fc regions. In some embodiments, the fusion protein comprises an amino acid sequence that has at least 85%, 90%, 95% or 99% sequence identity to SEQ ID NO: 38. In some embodiments, the fusion protein comprises an amino acid sequence of SEQ ID NO: 38.

In calculating percent sequence identity, the sequences being compared are aligned in a way that gives the largest match between the sequences. A computer program that can be used to determine percent identity is the GCG program package, which includes GAP (Devereux et al., (1984) Nucl. Acid Res. 12:387; Genetics Computer Group, University of Wisconsin, Madison, Wis.). The computer algorithm GAP can be used to align the two polypeptides or polynucleotides for which the percent sequence identity is to be determined. The sequences are aligned for optimal matching of their respective amino acid or nucleotide (the "matched span", as determined by the algorithm). A gap opening penalty (which is calculated as 3x the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm. In certain embodiments, a standard comparison matrix (see, Dayhoff et al., (1978) Atlas of Protein Sequence and Structure 5:345-352 for the PAM 250 comparison matrix; Henikoff et al., (1992) Proc. Natl. Acad. Sci. U.S.A. 9:10915-10919 for the BLOSUM 62 comparison matrix) is also used by the algorithm. Parameters that can be used for determining percent identity using the GAP program are the following:
Algorithm: Needleman et al., 1970, J. Mol. Biol. 48:443-453;
Comparison matrix: BLOSUM 62 from Henikoff et al., 1992, supra;
Gap Penalty: 12 (but with no penalty for end gaps)
Gap Length Penalty: 4
Threshold of Similarity: 0
Certain alignment schemes for aligning two amino acid sequences can result in matching of only a short region of the two sequences, and this small aligned region can have very high sequence identity even though there is no significant relationship between the two full-length sequences. Accordingly, the selected alignment method (e.g., the GAP program) can be adjusted if so desired to result in an alignment that spans at least 50 contiguous amino acids of the target polypeptide.

In some embodiments, the GDF15 molecule is FcΔ10(-)-(G4S)4-GDF15, FcΔ10(+)-(G4)-GDF15, FcΔ10(-)-GDF15(Δ3), FcΔ10(-)-GDF15(N3D), FcΔ10(-,CC)-GDF15(Δ3), FcΔ10(-,CC)-GDF15(N3D), FcΔ16(-,CC)-GDF15(Δ3/D5E), FcΔ16(-,CC)-GDF15(N3Q/D5E), FcΔ16(-)-GDF15(N3Q/D5E), FcΔ16(-)-(G4Q)4-GDF15, FcΔ16(-)-(G4Q)4-GDF15(N3Q), FcΔ16(-)-(G4Q)4-GDF15(N3Q/D5E), FcΔ16(-)-(G4S)2-GDF15(N3Q), FcΔ16(-)-(G4S)2- GDF15(N3Q/D5E), FcΔ16(-)-G4S-GDF15(N3Q), FcΔ16(-)-G4S-GDF15(N3Q/D5E), FcΔ16(-)-GDF15(N3Q), FcΔ10(-,L234A/L235A)-(G4Q)4-GDF15(N3Q), or FcΔ10(-,L234A/L235A)-(G4Q)4-GDF15(N3Q/D5E).

In some embodiments, the GDF15 molecule comprises the amino acid sequence of SEQ ID NO: 39, 40, 41, 42, 43, 44, 45, 46, 47, 48, 49, 50, 51, 52, 53, 54, 55, 56, or 57. In some embodiments, the GDF15 molecules comprises an amino acid sequence that has at least 85% sequence identity to SEQ ID NO: 39, 40, 41, 42, 43, 44, 45, 46, 47, 48, 49, 50, 51, 52, 53, 54, 55, 56, or 57. In some embodiments, the GDF15 molecules comprises an amino acid sequence that has at least 90% sequence identity to SEQ ID NO: 39, 40, 41, 42, 43, 44, 45, 46, 47, 48, 49, 50, 51, 52, 53, 54, 55, 56, or 57. In some embodiments, the GDF15 molecules comprises an amino acid sequence that has at least 95% sequence identity to SEQ ID NO: 39, 40, 41, 42, 43, 44, 45, 46, 47, 48, 49, 50, 51, 52, 53, 54, 55, 56, or 57. In some embodiments, the GDF15 molecules comprises an amino acid sequence that has at least 99% sequence identity to SEQ ID NO: 39, 40, 41, 42, 43, 44, 45, 46, 47, 48, 49, 50, 51, 52, 53, 54, 55, 56, or 57.

In some embodiments, the GDF15 molecule is a FcΔ10(-)-(G4S)4-GDF15, FcΔ10(+)-(G4)-GDF15, FcΔ10(-)-GDF15(Δ3), FcΔ10(-)-GDF15(N3D), FcΔ10(-,CC)-GDF15(Δ3), FcΔ10(-,CC)-GDF15(N3D), FcΔ16(-,CC)-GDF15(Δ3/D5E), FcΔ16(-,CC)-GDF15(N3Q/D5E), FcΔ16(-)-GDF15(N3Q/D5E), FcΔ16(-)-(G4Q)4-GDF15, FcΔ16(-)-(G4Q)4-GDF15(N3Q), FcΔ16(-)-(G4Q)4-GDF15(N3Q/D5E), FcΔ16(-)-(G4S)2-GDF15(N3Q), FcΔ16(-)-(G4S)2- GDF15(N3Q/D5E), FcΔ16(-)-G4S-GDF15(N3Q), FcΔ16(-)-G4S-GDF15(N3Q/D5E), FcΔ16(-)-GDF15(N3Q), FcΔ10(-,L234A/L235A)-(G4Q)4-GDF15(N3Q), or FcΔ10(-,L234A/L235A)-(G4Q)4-GDF15(N3Q/D5E) molecule that has at least 85%, 90%, 95% or 99% sequence identity to its Fc region and/or GDF15 region. For example, a FcΔ10(-)-(G4S)4-GDF15 molecule with at least 85%, 90%, 95% or 99% sequence identity to its Fc region and/or GDF15 region, includes a GDF15 molecule with an Fc region that has a ten-amino acid deletion of the hinge region and a negatively charged pair mutation, and has at least 85%, 90%, 95% or 99% sequence identity to SEQ ID NO: 26 and/or a GDF15 region that has at least 85%, 90%, 95% or 99% sequence identity to SEQ ID NO: 6. In another example, a FcΔ16(-)-(G4Q)4-GDF15(N3Q/D5E) molecule with at least 85%, 90%, 95% or 99% sequence identity to its Fc region and/or a GDF15 region, includes a GDF15 molecule with an Fc region that has a sixteen-amino acid deletion of the hinge region and a negatively charged pair mutation that has at least 85%, 90%, 95% or 99% sequence identity to SEQ ID NO: 30 and/or a GDF15 region that has at least 85%, 90%, 95% or 99% sequence identity to SEQ ID NO: 18. In yet another example, a FcΔ10(-,L234A/L235A)-(G4Q)4-GDF15(N3Q/D5E) molecule with at least 85%, 90%, 95% or 99% sequence identity to its Fc region and/or a GDF15 region, includes a GDF15 molecule with an Fc region that has a ten-amino acid deletion of the hinge region, a negatively charged pair mutation and leucine to alanine mutations at positions 234 and 235 and has at least 85%, 90%, 95% or 99% sequence identity to SEQ ID NO: 31 and/or a GDF15 region that has at least 85%, 90%, 95% or 99% sequence identity to SEQ ID NO: 18.

Also provided herein are dimers and tetramers comprising a GDF15 molecule provided herein. In one embodiment, the dimer comprises a GDF15-Fc fusion comprising the amino acid sequence of any one of SEQ ID NOs: 39-57. In some embodiments, a GDF15-Fc fusion comprising the amino acid sequence of SEQ ID NO: 39, 40, 41, 42, 43, 44, 45, 46, 47, 48, 49, 50, 51, 52, 53, 54, 55, 56 or 57 dimerizes with an Fc molecule comprising the amino acid sequence of SEQ ID NO: 32, 33, 34, 35, 36, or 37 (in which the C-terminal lysine is optional), such as shown in Table 6. For example, in some embodiments, the dimer is FcΔ10(-)-(G4S)4-GDF15: FcΔ10(+,K). In another embodiment, the dimer is FcΔ10(-,L234A/L235A)-(G4Q)4-GDF15(N3Q): FcΔ10(+,K,L234A/L235A). In yet another embodiment, the dimer is FcΔ10(-,L234A/L235A)-(G4Q)4-GDF15(N3Q):FcΔ10(+,K,L234A/L235A).

**Table 6: Dimers**

| **GDF15-Fc Fusion SEQ ID NO.** | **GDF15-Fc Fusion Designation** | **Fc Molecule SEQ ID NO.** | **Corresponding Fc Molecule Designation** |
|---|---|---|---|
| 39 | FcΔ10(-)-(G4S)4-GDF15 | 32 | FcΔ10(+K) |
| 40 | FcΔ10(+)-(G4)-GDF15 | 33 | FcΔ10(-,K) |
| 41 | FcΔ10(-)-GDF15(Δ3) | 32 | FcΔ10(+,K) |
| 42 | FcΔ10(-)-GDF15(N3D) | 32 | FcΔ10(+,K) |
| 43 | FcΔ10(-,CC)-GDF15(Δ3) | 34 | FcΔ10(+,K,CC) |
| 44 | FcΔ10(-,CC)-GDF15(N3D) | 34 | FcΔ10(+,K,CC) |
| 45 | FcΔ16(-,CC)-GDF15(Δ3/D5E) | 35 | FcΔ16(+,K,CC) |
| 46 | FcΔ16(-,CC)-GDF15(N3Q/D5E) | 35 | FcΔ16(+,K,CC) |
| 47 | FcΔ16(-)-GDF15(N3Q/D5E) | 36 | FcΔ16(+,K) |
| 48 | FcΔ16(-)-(G4Q)4-GDF15 | 36 | FcΔ16(+,K) |
| 49 | FcΔ16(-)-(G4Q)4-GDF15(N3Q) | 36 | FcΔ16(+,K) |
| 50 | FcΔ16(-)-(G4Q)4-GDF15(N3Q/D5E) | 36 | FcΔ16(+,K) |
| 51 | FcΔ16(-)-(G4S)2-GDF15(N3Q) | 36 | FcΔ16(+,K) |
| 52 | FcΔ16(-)-(G4S)2- GDF15(N3Q/D5E) | 36 | FcΔ16(+,K) |
| 53 | FcΔ16(-)-G4S- GDF15(N3Q) | 36 | FcΔ16(+,K) |
| 54 | FcΔ16(-)-G4S-GDF15(N3Q/D5E) | 36 | FcΔ16(+,K) |
| 55 | FcΔ16(-)-GDF15(N3Q) | 36 | FcΔ16(+,K) |
| 56 | FcΔ10(-L234A/L235A)-(G4Q)4-GDF15(N3Q) | 37 | FcΔ10(+,K,L234A/L235A) |
| 57 | FcΔ10(-L234A/L235A)-(G4Q)4-GDF15(N3Q/D5E) | 37 | FcΔ10(+,K,L234A/L235A) |

In one embodiment, a GDF15-Fc fusion comprising the amino acid sequence of SEQ ID NO: 39 dimerizes with an Fc molecule comprising SEQ ID NO: 32 (C-terminal lysine optional). In another embodiment, a GDF15-Fc fusion comprising the amino acid sequence of SEQ ID NO: 40 dimerizes with an Fc molecule comprising SEQ ID NO: 33 (C-terminal lysine optional). In another embodiment, a GDF15-Fc fusion comprising the amino acid sequence of SEQ ID NO: 41 dimerizes with an Fc molecule comprising SEQ ID NO: 32 (C-terminal lysine optional). In another embodiment, a GDF15-Fc fusion comprising the amino acid sequence of SEQ ID NO: 42 dimerizes with an Fc molecule comprising SEQ ID NO: 32 (C-terminal lysine optional). In another embodiment, a GDF15-Fc fusion comprising the amino acid sequence of SEQ ID NO: 43 dimerizes with an Fc molecule comprising SEQ ID NO: 34 (C-terminal lysine optional). In another embodiment, a GDF15-Fc fusion comprising the amino acid sequence of SEQ ID NO: 44 dimerizes with an Fc molecule comprising SEQ ID NO: 34 (C-terminal lysine optional). In another embodiment, a GDF15-Fc fusion comprising the amino acid sequence of SEQ ID NO: 44 dimerizes with an Fc molecule comprising SEQ ID NO: 34 (C-terminal lysine optional). In another embodiment, a GDF15-Fc fusion comprising the amino acid sequence of SEQ ID NO: 45 dimerizes with an Fc molecule comprising SEQ ID NO: 35 (C-terminal lysine optional). In another embodiment, a GDF15-Fc fusion comprising the amino acid sequence of SEQ ID NO: 46 dimerizes with an Fc molecule comprising SEQ ID NO: 35 (C-terminal lysine optional). In another embodiment, a GDF15-Fc fusion comprising the amino acid sequence of SEQ ID NO: 47 dimerizes with an Fc molecule comprising SEQ ID NO: 36 (C-terminal lysine optional). In another embodiment, a GDF15-Fc fusion comprising the amino acid sequence of SEQ ID NO: 48 dimerizes with an Fc molecule comprising SEQ ID NO: 36 (C-terminal lysine optional). In another embodiment, a GDF15-Fc fusion comprising the amino acid sequence of SEQ ID NO: 49 dimerizes with an Fc molecule comprising SEQ ID NO: 36 (C-terminal lysine optional). In another embodiment, a GDF15-Fc fusion comprising the amino acid sequence of SEQ ID NO: 50 dimerizes with an Fc molecule comprising SEQ ID NO: 36 (C-terminal lysine optional). In another embodiment, a GDF15-Fc fusion comprising the amino acid sequence of SEQ ID NO: 51 dimerizes with an Fc molecule comprising SEQ ID NO: 36 (C-terminal lysine optional). In another embodiment, a GDF15-Fc fusion comprising the amino acid sequence of SEQ ID NO: 52 dimerizes with an Fc molecule comprising SEQ ID NO: 36 (C-terminal lysine optional). In another embodiment, a GDF15-Fc fusion comprising the amino acid sequence of SEQ ID NO: 53 dimerizes with an Fc molecule comprising SEQ ID NO: 36 (C-terminal lysine optional). In another embodiment, a GDF15-Fc fusion comprising the amino acid sequence of SEQ ID NO: 54 dimerizes with an Fc molecule comprising SEQ ID NO: 36 (C-terminal lysine optional). In another embodiment, a GDF15-Fc fusion comprising the amino acid sequence of SEQ ID NO: 55 dimerizes with an Fc molecule comprising SEQ ID NO: 36 (C-terminal lysine optional). In another embodiment, a GDF15-Fc fusion comprising the amino acid sequence of SEQ ID NO: 56 dimerizes with an Fc molecule comprising SEQ ID NO: 37 (C-terminal lysine optional). In another embodiment, a GDF15-Fc fusion comprising the amino acid sequence of SEQ ID NO: 57 dimerizes with an Fc molecule comprising SEQ ID NO: 37 (C-terminal lysine optional).

In some embodiments, the dimers form tetramers. For example, the dimers in Table 6 can form tetramers. In some embodiments, the tetramers are formed form the same dimers. In some embodiments, two dimers of FcΔ10(-)-(G4S)4-GDF15:FcΔ10(+,K); FcΔ10(+)-(G4)-GDF15:FcΔ10(-,K); FcΔ10(-)-GDF15(Δ3):FcΔ10(+,K); FcΔ10(-)-GDF15(N3D):FcΔ10(+,K); FcΔ10(-,CC)-GDF15(Δ3):FcΔ10(+,K,CC); FcΔ10(-,CC)-GDF15(N3D):FcΔ10(+,K,CC); FcΔ16(-,CC)-GDF15(Δ3/D5E):FcΔ16(+,K,CC); FcΔ16(-,CC)-GDF15(N3Q/D5E):FcΔ16(+,K,CC); FcΔ16(-)-GDF15(N3Q/D5E):FcΔ16(+,K); FcΔ16(-)-(G4Q)4-GDF15:FcΔ16(+,K); FcΔ16(-)-(G4Q)4-GDF15(N3Q):FcΔ16(+,K); FcΔ16(-)-(G4Q)4-GDF15(N3Q/D5E):FcΔ16(+,K); FcΔ16(-)-(G4S)2-GDF15(N3Q):FcΔ16(+,K); FcΔ16(-)-(G4S)2- GDF15(N3Q/D5E):FcΔ16(+,K); FcΔ16(-)-G4S- GDF15(N3Q):FcΔ16(+,K); FcΔ16(-)-G4S-GDF15(N3Q/D5E): FcΔ16(+,K); FcΔ16(-)-GDF15(N3Q): FcΔ16(+,K); FcΔ10(-,L234A/L235A)-(G4Q)4-GDF15(N3Q):FcΔ10(+,K,L234A/L235A); or FcΔ10(-,L234A/L235A)-(G4Q)4-GDF15(N3Q/D5E):FcΔ10(+,K,L234A/L235A) form a tetramer, such as through the dimerization of the two GDF15 regions.

In some embodiments, the GDF15 molecule is a GDF15 protein fused to human serum albumin (HSA) or a functional variant thereof, optionally through a linker. In some embodiments, the GDF15 molecule is a GDF15 molecule or compound, such as a mutein and/or fusion protein, or complex or dimer comprising a GDF15 molecule or compound, such as disclosed in PCT publication WO 2014/120619A2, WO 2013/148117 A1, WO 2016/018931 A1, WO 2016/069921 A1, WO 2015/197446 A1, WO 2017/202936 A1, WO 2018/215525 A1, WO 2017/196647 A1, WO 2020/104948 A1, WO 2019/195091 A1, WO 2015/198199 A1, WO 2017/109706 A1, WO 2015/200078 A1.

Also provided herein are host cells comprising the nucleic acids and vectors for producing the GDF15 and Fc molecules disclosed herein. In various embodiments, the vector or nucleic acid is integrated into the host cell genome, which in other embodiments the vector or nucleic acid is extra-chromosomal.

Recombinant cells, such as yeast, bacterial (*e.g., E. coli*)*,* and mammalian cells (*e.g.,* immortalized mammalian cells) comprising such a nucleic acid, vector, or combinations of either or both thereof are provided. In various embodiments, cells comprising a non-integrated nucleic acid, such as a plasmid, cosmid, phagemid, or linear expression element, which comprises a sequence coding for expression of a GDF15 molecule and/or an Fc molecule. In some embodiments, the cell comprises a nucleic acid for producing a GDF15 molecule and another cell comprises a nucleic acid for producing an Fc molecule for dimerization with the GDF15 molecule (e.g., a vector for encoding a GDF15 molecule in one cell and a second vector for encoding an Fc molecule in a second cell). In other embodiments, a host cell comprises a nucleic acid for producing a GDF15 molecule and an Fc molecule (e.g., a vector that encodes both molecules). In another embodiment, a host cell comprises a nucleic acid for producing a GDF15 molecule and another nucleic acid for producing an Fc molecule (e.g., two separate vectors, one that encodes a GDF15 molecule and one that encodes an Fc molecule, in a single host cell)

A vector comprising a nucleic acid sequence encoding a GDF15 molecule and/or an Fc molecule can be introduced into a host cell by transformation or by transfection, such as by methods known in the art.

A nucleic acid encoding a GDF15 molecule can be positioned in and/or delivered to a host cell or host animal via a viral vector. A viral vector can comprise any number of viral polynucleotides, alone or in combination with one or more viral proteins, which facilitate delivery, replication, and/or expression of the nucleic acid of the invention in a desired host cell. The viral vector can be a polynucleotide comprising all or part of a viral genome, a viral protein/nucleic acid conjugate, a virus-like particle (VLP), or an intact virus particle comprising viral nucleic acids and a nucleic acid encoding a polypeptide comprising a GDF15 region. A viral particle viral vector can comprise a wild-type viral particle or a modified viral particle. The viral vector can be a vector which requires the presence of another vector or wild-type virus for replication and/or expression (e.g., a viral vector can be a helper-dependent virus), such as an adenoviral vector amplicon. Suitable viral vector particles in this respect, include, for example, adenoviral vector particles (including any virus of or derived from a virus of the adenoviridae), adeno-associated viral vector particles (AAV vector particles) or other parvoviruses and parvoviral vector particles, papillomaviral vector particles, flaviviral vectors, alphaviral vectors, herpes viral vectors, pox virus vectors, retroviral vectors, including lentiviral vectors.

A GDF15 molecule can be isolated using standard protein purification methods. A polypeptide comprising a GDF15 region can be isolated from a cell that has been engineered to express a polypeptide comprising a GDF15 region, for example a cell that does not naturally express native GDF15. Protein purification methods known in the art can be employed to isolate GDF15 molecules, as well as associated materials and reagents. Methods of purifying a GDF15 molecule are also provided in the Examples herein. Additional purification methods that may be useful for isolating GDF15 molecules can be found in references such as Bootcov MR, 1997, Proc. Natl. Acad. Sci. USA 94:11514-9*,* Fairlie WD, 2000, Gene 254: 67-76*.*

Pharmaceutical compositions comprising a GDF15 molecule (and optionally, an Fc molecule, such as a dimer or tetramer disclosed herein) are also provided. Such polypeptide pharmaceutical compositions can comprise a therapeutically effective amount of a GDF15 molecule in admixture with a pharmaceutically or physiologically acceptable formulation agent or carrier selected for suitability with the mode of administration. The pharmaceutically or physiologically acceptable formulation agent can be one or more formulation agents suitable for accomplishing or enhancing the delivery of a GDF15 molecule into the body of a human or non-human subject. Pharmaceutically acceptable substances such as wetting or emulsifying agents, preservatives or buffers, which enhance the shelf life or effectiveness of the GDF15 molecule can also act as, or form a component of, a formulation carrier. Acceptable pharmaceutically acceptable carriers are preferably nontoxic to recipients at the dosages and concentrations employed. The pharmaceutical composition can contain formulation agent(s) for modifying, maintaining, or preserving, for example, the pH, osmolarity, viscosity, clarity, color, isotonicity, odor, sterility, stability, rate of dissolution or release, adsorption, or penetration of the composition.

The effective amount of pharmaceutical composition comprising a GDF15 molecule which is to be employed therapeutically will depend, for example, upon the therapeutic context and objectives. One skilled in the art will appreciate that the appropriate dosage levels for treatment will thus vary depending, in part, upon the molecule delivered, the indication for which a GDF15 molecule is being used, the route of administration, and the size (body weight, body surface, or organ size) and condition (the age and general health) of the subject. The frequency of dosing will depend upon the pharmacokinetic parameters of the GDF15 molecule in the formulation being used.

The route of administration of the pharmaceutical composition can be orally; through injection by intravenous, intraperitoneal, intracerebral (intraparenchymal), intracerebroventricular, intramuscular, intraocular, intraarterial, intraportal, or intralesional routes; by sustained release systems (which may also be injected); or by implantation devices. Where desired, the compositions can be administered by bolus injection or continuously by infusion, or by an implantation device. The composition can also be administered locally via implantation of a membrane, sponge, or other appropriate material onto which the desired molecule has been absorbed or encapsulated. Where an implantation device is used, the device can be implanted into any suitable tissue or organ, and delivery of the desired molecule can be via diffusion, timed-release bolus, or continuous administration.

According to claim 1, a GDF15 molecule can be used to treat, a heart condition, which is heart failure with preserved ejection fraction (HFpEF).

Also provided is a GDF15 molecule for use in a method of treating a subject with cardiometabolic syndrome, the method comprising administering the GDF15 molecule to the subject. In some embodiments, a subject with CMS is obese. In some embodiments, a subject with CMS has impaired metabolism, pancreatic dysfunction, hypertension, diastolic dysfunction (DD), HFpEF, decreased exercise capacity, or renal dysfunction. For example, the subject may have an increase in body weight or heart weight, or decreased adiponectin, as compared to a non-obese subject. A subject may have an increase in glucose, triglycerides, cholesterol, or insulin levels as compared to a subject without impaired metabolism. A subject may have levels of c-peptide, proinsulin, amylin or glucagon that differs from a subject without pancreatic dysfunction. A subject with hypertension, DD, or HFpEF may have increased levels of aldosterone, FABP3, IL-16, or ST2, as compared to a subject without hypertension, DD, or HFpEF. In some embodiments, a subject with hypertension, DD, or HFpEF has a decreased heart rate, increased Tau level, increased E/E' ratio (abnormal LV filling pressure may be estimated noninvasively by E/E' >15), or increased isovolumic relaxation time (IVRT), as compared to a subject without hypertension, DD, or HFpEF. In some embodiments, the subject with hypertension, DD, or HFpEF has the same left ventricular ejection fraction (LVEF) percentage as a subject without hypertension, DD, or HFpEF. A subject with decreased exercise capacity may have a decrease in running distance, time (e.g., time to exhaustion), or peak VO2 as compared to a subject without decreased exercise capacity. In some embodiments, a subject with renal dysfunction has an increase in NGAL, KIM-1, or clusterin levels as compared to a subject without renal dysfunction.

In some subjects with CMS, after treatment with a GDF15 molecule, the subject has reduced CMS. For example, the subject may have improved metabolism, pancreatic function, hypertension, diastolic function, exercise capacity, or renal function. For example, the subject may have a decrease in body weight or heart weight, or increased adiponectin, as compared to prior to treatment with a GDF15 molecule. A subject may have a decrease in glucose, triglycerides, cholesterol, or insulin levels as compared to prior to treatment with a GDF15 molecule. A subject treated with a GDF15 molecule may have levels of c-peptide, proinsulin, amylin or glucagon that is similar to that of a subject without pancreatic dysfunction. The subject may have decreased levels of aldosterone, FABP3, IL-16, or ST2, as compared to prior to treatment with a GDF15 molecule. In some embodiments, a subject has an increase in heart rate, decrease in Tau level, decrease in E/E' ratio, or decrease in IVRT, as compared to prior to treatment with a GDF15 molecule. In some embodiments, a subject has an increase in running distance, time (e.g., time to exhaustion), or peak VO2 as compared to a prior to treatment with a GDF15 molecule. In some embodiments, the subject has a decrease in NGAL, KIM-1, or clusterin levels as compared to prior to GDF15 treatment.

In some embodiments, after treatment with the GDF15 molecule, the subject has a decreased heart weight, improved exercise capacity, or change in the level of adiponectin, sE-selectin, sICAM, Myl3, osteopontin, TIMP1, VEGF, or vWF, after treatment with the GDF15 molecule. In some embodiments, the level of adiponectin is increased. In some embodiments, the level of sE-selectin, sICAM, Myl3, osteopontin, TIMP1, VEGF, vWF, or any combination thereof is decreased. In yet other embodiments, the subject has decreased heart to brain weight ratio, left ventricle (LV) mass, cardiac output, stroke volume, or % ejection fraction echocardiographic, after treatment with the GDF15 molecule.

The administration can be performed such as by IV injection, intraperitoneal (IP) injection, subcutaneous injection, intramuscular injection, or orally in the form of a tablet or liquid formation. A therapeutically effective dose of a GDF15 molecule will depend upon the administration schedule, the unit dose of agent administered, whether the GDF15 molecule is administered in combination with other therapeutic agents, the immune status and the health of the recipient. A therapeutically effective dose is an amount of a GDF15 molecule that elicits a biological or medicinal response in a tissue system, animal, or human being sought by a researcher, medical doctor, or other clinician, which includes alleviation or amelioration of the symptoms of the disease or disorder being treated, *i.e.,* an amount of a GDF15 molecule that supports an observable level of one or more desired biological or medicinal response, such as decrease in body weight or heart weight; increase in adiponectin; decrease in glucose, triglycerides, cholesterol, or insulin levels; changes in levels of c-peptide, proinsulin, amylin or glucagon; decrease in level of aldosterone, FABP3, IL-16, or ST2; increase in heart rate; decrease in Tau level; decrease in E/E' ratio; decrease in IVRT; increase in running distance, time (e.g., time to exhaustion), or peak VO2; decrease in NGAL, KIM-1, or clusterin level; decrease in level of sE-selectin, sICAM, Myl3, osteopontin, TIMP1, VEGF, or vWF; decrease in heart to brain weight ratio; decrease in left ventricle (LV) mass; decrease in cardiac output; decrease in stroke volume, or decrease in % ejection fraction. A therapeutically effective dose of a GDF15 molecule can also vary with the desired result.

Also illustrated herein is a method comprising measuring a baseline level of one or more of body weight; heart weight; adiponectin level; decrease in glucose, triglycerides, cholesterol, or insulin levels; c-peptide, proinsulin, amylin or glucagon level; aldosterone, FABP3, IL-16, or ST2 level; heart rate; Tau level; E/E' ratio; IVRT; running distance, time (e.g., time to exhaustion) or peak VO2; NGAL, KIM-1, or clusterin level; sE-selectin, sICAM, Myl3, osteopontin, TIMP1, VEGF, or vWF level; heart to brain weight ratio; left ventricle (LV) mass; cardiac output; stroke volume; and % ejection fraction; in a subject, administering a pharmaceutical composition comprising a GDF15 molecule to the subject, and after a desired period of time, measuring the level of one or more of body weight; heart weight; adiponectin level; decrease in glucose, triglycerides, cholesterol, or insulin levels; c-peptide, proinsulin, amylin or glucagon level; aldosterone, FABP3, IL-16, or ST2 level; heart rate; Tau level; E/E' ratio; IVRT; running distance, time (e.g., time to exhaustion) or peak VO2; NGAL, KIM-1, or clusterin level; sE-selectin, sICAM, Myl3, osteopontin, TIMP1, VEGF, or vWF level; heart to brain weight ratio; left ventricle (LV) mass; cardiac output; stroke volume; and % ejection fraction; in the subject. The two levels can then be compared to determine the relative change in the subject. Depending on the outcome of that comparison another dose of the pharmaceutical composition can be administered to achieve a desired level of one or more metabolically-relevant compound.

A GDF15 molecule (and optionally, its corresponding Fc molecule) can be administered in combination with another therapeutic agent, such as an agent that decrease in body weight or heart weight; increase in adiponectin; decrease in glucose, triglycerides, cholesterol, or insulin levels; changes in levels of c-peptide, proinsulin, amylin or glucagon; decrease in level of aldosterone, FABP3, IL-16, or ST2; increase in heart rate; decrease in Tau level; decrease in E/E' ratio; decrease in IVRT; increase in running distance, time (e.g., time to exhaustion), or peak VO2; decrease in NGAL, KIM-1, or clusterin level; decrease in level of sE-selectin, sICAM, Myl3, osteopontin, TIMP1, VEGF, or vWF; decrease in heart to brain weight ratio; decrease in left ventricle (LV) mass; decrease in cardiac output; decrease in stroke volume, or decrease in % ejection fraction.

The detailed description and following examples illustrate the present invention and are not to be construed as limiting the present invention thereto.

### EXAMPLES

The following examples, including the experiments conducted and results achieved, are provided for illustrative purposes only.

### Example 1: In vivo study design for characterization of ZSF1 obese rat as preclinical model of human cardiometabolic syndrome

Eighteen-week-old ZSF1 male Lean (strain 379, n=14) and ZSF1 Obese (strain 378, n=14) rats were purchased from Charles Rivers Laboratories (Kingston, NY, USA) and single-housed at an accredited facility. After 2 weeks of acclimation both groups (n=8 per group) were subjected to the blood collection via tail vein, echocardiography, hemodynamic assessment and to evaluation of exercise endurance (time and distance) using a treadmill regimen. The separate cohort from the same batch of acclimated ZSF1 obese (n=6) and lean (n=6) rats was subjected to the heart isolation, RNA extraction and expression analysis of RNAseq results.

### Example 2: In vivo study design for the evaluation of GDF15 treatment effect on cardiometabolic syndrome specific biomarkers in ZSF1 obese rats

Twenty-week-old ZSF1 obese male rats (strain 378, n=30) were purchased from (Charles Rivers Laboratories Kingston, NY, USA) single-housed at an accredited facility and acclimated for 2 weeks. At 22 weeks of age, the baseline blood collection (for metabolic biomarkers evaluation) and body weight assessment were performed for all the animals. Twenty-four hours later rats were randomized into two groups and injected subcutaneously once a week for 12 weeks with either A5.2Su buffer (Vehicle group, n=15) or 1.5 mg/kg of FcΔ10(-)-(G4S)4-GDF15 (SEQ ID NO: 39, see PCT Application No. PCT/US19/26369) and FcΔ10(+,K) (SEQ ID NO: 32) (FC-GDF15 group, n=15). For the duration of a study all the rats were subjected to the weekly: (1) blood collection, (2) food intake and (3) bodyweight assessments. At the end of the study rats from both treatment groups were subjected to echocardiography, hemodynamic assessment and to evaluation of exercise endurance (time and distance) using a treadmill regimen.

### Example 3: Evaluation of cardiovascular, kidney injury and metabolic biomarkers in serum/plasma

Prior to the blood collection animals were fasted for 4 hours. The first 0.5 ml aliquot of whole blood was collected from the tail vein into a serum separator tubes (Microtainer; Becton Dickinson; Franklin Lakes, NJ, USA) and the second 0.5 ml aliquot of whole blood was in EDTA plasma separation tube (Microtainer; Becton Dickinson; Franklin Lakes, NJ, USA). Separated serum/plasma was aliquoted and stored at -80°C. Blood glucose levels were measured using AlphaTrak 2 glucose strip (Abbott Laboratories, Lake Bluff, Illinois, USA). Blood insulin was evaluated by rat insulin ELISA kit (Alpco, Salem, NH, USA). Serum triglyceride and cholesterol levels were measured weekly by triglyceride quantitation kits (Fisher Diagnostics, Middletown, VA, USA). Systemic levels of GDF15 (R&D Systems, Minneapolis, MN, US) and proinsulin (Mercodia, Uppsala, Sweden) in serum/plasma of 20-week-old ZSF1 male rats were evaluated by commercial ELISAs. The limited array of metabolic hormones in circulation (Amylin (active), C-Peptide 2, Ghrelin (active), GIP (total), GLP-1 (active), Glucagon, IL-6, Leptin, PP, PYY) was assessed in serum/plasma collected from ZSF1 obese and lean cohorts at the age of 20 weeks by rat-specific MILLIplex kit (Millipore; Billerica, MA, USA). Kidney injury markers KIM-1 and NGAL were evaluated in serum from 20-week-old ZSF1 obese and lean cohorts by multiplex MILLIplex kits (Millipore; Billerica, MA, USA). Serum NT-proBNP and rat cardiac injury markers (FABP3, Myl3) were measured by using rat-specific single-plex or multi-plex commercial assays from Meso Scale Diagnostics (Rockville, MD, USA). Systemic levels of vascular markers were evaluated at the end of FC-GDF15 or Vehicle treatment of ZSF1 obese rats by using multiplex MILLIplex kits (Millipore: Billerica, MA, USA): Rat Vascular injury panels included VEGF, MCP1, TIMP1, TNFa, vWF, Adiponectin, sE-Selectin and sICAM1. Systemic aldosterone was measured by enzyme immunoassay (m/r/h Aldosterone ELISA, Enzo Life Sciences, Farmingdale, NY, USA) and serum ostepontin - by rat Single plex kit (Rat Millipore; Billerica, MA, USA). All the assays were performed in accordance with manufacturer protocols.

### Example 4: RNA isolation and sequencing analysis of left heart

At scheduled necropsy, left atria and left ventricle of the heart were washed briefly in RNase-free saline, placed in cryo-tubes, snap-frozen in liquid nitrogen, and stored at -80°C. Samples were subjected to dry pulverization before being homogenized in buffer (350 µL of Qiagen RLT buffer with 10% BME; Qiagen [Germantown, MD, USA]). The homogenate was transferred to an RNase-free 1.5 mL centrifuge tube, after which 95 µL RNase-free water and 5 µL of a 20 mg/mL Proteinase K solution were added. Samples were incubated at 55°C for 10 minutes, centrifuged and the supernatant collected. RNA was then extracted using RNeasy Micro Kit (Qiagen) with on-column DNase treatment (Qiagen) according to manufacturer's instructions. RNA concentration and integrity were assessed using a Bioanalyzer (Agilent [Santa Clara, CA, USA]). Samples with ≥ 80 ng total RNA that had RNA integrity numbers ≥ 7 were used for sequencing (Supplemental Table IV). Principal component analysis was performed on log2 (Fragments per Kilobase per Million Quantile normalized [FPKQ]) values for each time point using OmicSoft's Array Studio software (www.omicsoft.com/array-studio.php; Oshell.exe v10.0).

### Example 5: Differential expression and pathway analysis

RSEM was used to calculate gene expression levels and DESEQ2 was used to identify differentially expressed genes (DEGs) by BH adjusted p value less than 0.05 and fold change more than 1.5. The analysis of the genes that have human homologue and are abundant in human heart was based on GTEX data. All the DEGs and heart abundant DEGs were further annotated by ingenuity pathway analysis (IPA). Metabolic and cardiac signaling pathway enrichment was done on the significantly differentially expressed genes by adjust p-value cutoff of 0.05 through the use of IPA (QIAGEN, Redwood City, CA, US). Gene expression enrichment in cardiac diseases was analyzed by using Medical Subject Headings (MeSH) terms with meshes R package (Yu G. Using meshes for MeSH term enrichment and semantic analyses. Bioinformatics, 2018, 34(21), 3766-3767). Dotplots were generated with enrichplot R package (Yu G. Enrichplot: Visualization of Functional Enrichment Result. R package version 1.4.0. https://github.com/GuangchuangYu/enrichplot, 2019)

### Example 6: Echocardiography

Non-invasive echocardiograms were obtained on anesthetized rats (isoflurane, 3% induction, 1.5% maintenance) using a Vevo 2100 imaging system. Animals were shaved, placed on a stage, and a thermo-couple probe was used to assess body temperature and to adjust the temperature of the platform to maintain normothermia. Sonography gel was applied on the thorax. Two-dimensional targeted B-mode and M-mode imaging was obtained from the long-axis and short-axis view at the level of the papillary muscle. Coronary flow was measured by Pulsed-Wave (PW) Doppler and movement of the LV wall measured by Tissue Doppler by placing the probe to the chest and focusing image on region of the ventricular wall to achieve an apical four chamber view. Animals were subsequently wiped clean of sonography gel, allowed to achieve consciousness, and returned to their home cage.

### Example 7: Invasive hemodynamic assessment

Animals underwent general anesthesia using ketamine/diazepam (IP) injection (80 to 100 mg/kg ketamine; 5 to 10 mg/kg diazepam) followed by a ketamine boost (10 to 20 mg/kg) as necessary to maintain anesthesia plane. Anesthetized animals had the surgical sites shaved with a small animal shear. Animals were then placed on a heated surgical platform, and body temperature was monitored and maintained throughout the study via a rectal probe. Arterial pressure was measured via femoral artery catheterization (SPR-839, Millar). An incision in the medial aspect of the leg was made and the fat overlaying the femoral vessels and femoral nerve in the area between the abdominal wall and the upper leg was gently teased apart using forceps or Q-tips. The fascia overlying the artery, nerve and vein was removed. The artery was isolated and ligated by a distal suture and a loose tie was held in place using a hemostat. A microvessel clip or suture was placed on the artery near the abdominal wall and a small incision was made into the vessel using Vannas micro-scissors or a 25-gauge needle. The clip was released by one hand, and the Millar catheter was advanced quickly into the femoral artery, followed by the tightening of the proximal suture to prevent blood loss. Pressure was then recorded. Left ventricular pressure and volume were measured via carotid artery catheterization (SPR-838, Millar). The right carotid artery was isolated, and a suture was tied around the vessel approximately 0.25 cm below the skull base to disrupt blood flow. A suture was placed around the artery near the rib cage and held in place using a hemostat. A small incision was made into the vessel using Vannas micro-scissors or a 25-gauge needle. The clip was released by one hand, and the Millar catheter was advanced quickly into the carotid artery, followed by the tightening of the proximal suture to prevent blood loss. The catheter was advanced until it entered the left ventricle for pressure-volume loop measurement. Animals were euthanized under anesthesia after completion of the data acquisition.

### Example 8: Treadmill assessment

Rats were subjected to evaluation of exercise endurance (time and distance) using a treadmill regimen. Endurance exercise performance were estimated using two parameters: run duration (min.) and distance (m). Oxygen consumption, an indicator of exercise capacity, was measured by monitoring the O2 concentration of expired air. Peak chamber oxygen decrement, which usually occurs just prior to exhaustion, was used to calculate and define the animal's peak VO2. The contribution of anaerobic metabolism to overall energy production during exercise was estimated by calculating the respiratory exchange ratio (RER), the ratio of VO2 to VCO2 (the amount of CO2 within the chamber just prior to exhaustion). Rats were initially familiarized to the motorized rodent treadmill (Columbus Instruments, Columbus, OH) for 5 days before completing the exercise performance test. Rats were placed on the treadmill and allowed to adapt to the surroundings for 2-5 minutes before starting. Familiarization runs consisted of 10 minutes of running on an incline of 10 degrees at a speed of 12 m/min. For the treadmill challenge, rats were placed on the treadmill and allowed to adapt to the surroundings for 2-5 minutes before starting. The treadmill was initiated at a speed of 8.5/min with a zero-degree incline. After 3 minutes, the speed and incline were raised up to 10m/min. The incline was subsequently increased progressively by 2.5m/min every 3 min. The incline was progressively increased by 5 degrees every 9 min. to a maximum of up to 30 degrees. Exercise continued until exhaustion, which is defined as the inability to maintain running speed despite repeated contact with the electric grid (3 shocks in less than 15 seconds).

### Example 9: Statistical analysis

Results for serum protein biomarkers are expressed as mean ± SEM. Differences between the two groups were examined between ZSF1 Lean and ZSF1 OB rats of the same age or at the end of the hGDF15 treatment (wk12) between Vehicle-treated and hGDF15-treated ZSF1 OB rats. Unpaired Student's *t* test was performed to evaluate the statistical significance between the groups. A value of p < 0.05 was used to determine statistical significance.

The RNAseq data set from each cohort was analyzed separately, genes with FPKQ ≥ 1 in ≥ 5 samples in the pairwise group comparison were selected for further statistical testing. Differential expression analysis was performed using DESeq2 version 1.10.1 for group comparison (Love, M. I., et al. (2014). "Moderated estimation of fold change and dispersion for RNA-seq data with DESeq2." Genome Biol 15(12): 550). For a given comparison, raw reads counts for genes were compared between the 2 conditions using DESeq2 following a negative binomial distribution, and gene expression fold changes were calculated using normalized counts. Genes with Benjamini-Hochberg corrected p-value < 0.05 and fold change between the two conditions ≥ 1.5 or ≤ 0.5 were selected as significantly differentially expressed genes. Stars (*) indicate significance: * p <0.05, ** p <0.01, *** p <0.001, **** p <0.0001.

### Example 10: Gene Models Used for Alignment and Quantification

RNA-Seq alignment and quantification utilized slightly modified versions of the gene model, genome build 38 using GENCODE v24 annotation, curated by OmicSoft (Cary, NC, USA). The modifications to the gene models imposed a more stringent definition of the term "gene", requiring that all of the gene's transcripts reside on the same chromosome and strand, and that the distance between a gene's transcripts not span more than 10 kilobases that is not covered by any (un-spliced) transcript. Genes that violated this definition were split into multiple genes to comply with these criteria, and the gene names were appended with a suffix indicating the strand, chromosome name, and transcript bundle number.

### Example 11: Read Filtering and Trimming

All read files were pre-processed to remove poor quality reads and to trim poor quality 3' bases. First, any read pair containing a read that failed the Illumina quality filter was removed. Next, all reads were trimmed from the 3' terminus until either (1) the read length was reduced to 40 bp, or (2) less than 2 of the bases in the 5 base window at the 3' terminus of the read had a quality scores value below 3. This trimming included a final polishing step in which the base at the 3' terminus was trimmed if the read length exceeded 40 bp and the quality score of the base was below 3.

### Example 12: Alignment and Quantification

The filtered and trimmed reads were aligned and quantified using OmicSoft's Array Studio software (www.omicsoft.com/array-studio.php; Oshell.exe v10.0). This software compared favorably to other commonly used tools (eg Tophat/Cufflinks, MMSEQ) in internal benchmark comparisons. The alignment algorithm is documented in an OmicSoft whitepaper (www.omicsoft.com/downloads/whitepaper/OmicsoftAligner.pdf), and the gene/transcript quantification module is an OmicSoft implementation of the RSEM algorithm (Li, B. and C. N. Dewey (2011). "RSEM: accurate transcript quantification from RNA-Seq data with or without a reference genome." BMC Bioinformatics 12: 323).

### Example 13: Differential Expression Analysis

Genes with critical issues on gene models, including duplicated regions, high GC content and overlapping transcripts were removed from statistical analysis. RNA-Seq data set from each time point was analyzed separately. For each time point, genes expressed at FPKQ>=1 in at least 5 samples were selected for further statistical test. Differential Expression Analysis was performed using DESeq2 version 1.18.1 (Love, M. I., et al. (2014)). Raw read counts from the selected genes were compared using R Bioconductor package DESeq2 following Negative Binomial distribution. Gene expression Fold Changes were calculated using normalized counts. Genes with BH corrected p value <0.05 and Fold Change >=1.5 or <=2/3 were selected as significantly differentially expressed genes.

### Example 14: Expression Normalization

The gene- and transcript-level expression of each RNA-Seq sample was reported by Oshell as an FPKM (Fragments per Kilobase per Million sequenced) value. FPKM is an expression measurement based on the count of library fragments that map to a given feature (gene or transcript) normalized for feature length and the number of fragments sequenced. These values were adjusted on a per-sample basis using 70^{th} percentile normalization, yielding a final expression metric which is referred to as "FPKQ".

### Example 15: ZSF1 obese rats exhibit obesity and impaired metabolism

ZSF1 obese male rats at the age of 20 weeks exhibited impaired markers of metabolism as shown in **Table 7** and **Figure 1****.**

**Table 7: Metabolic and renal biomarkers in serum/plasma^ of 20-week-old ZSF1 Lean and ZSF1 OB male rats**

| **Biomarker** | **Serum concentration (Mean ± SEM)** | | **P value** |
|---|---|---|---|
| | **ZSF1 Lean** | **ZSF1 OB** | |
| Cholesterol (mg/dl) | 64.6 ± 2.0 | 271.1 ± 13.4 | <0.0001 |
| Triglycerides (mg/dl) | 118.5 ± 7.6 | 3239 ± 163 | <0.0001 |
| BUN (mg/dl) | 20.5 ± 0.5 | 23.5 ± 1.0 | 0.0178 |
| LDL (mg/dl) | 11.2 ± 0.3 | 12.0 ± 0.6 | 0.2749 |
| Glucose (mg/dl) | 110.0 ± 3.6 | 350.6 ± 29.4 | <0.0001 |
| Insulin^ (ng/ml) | 2.9 ± 0.6 | 11.1 ± 2.9 | 0.0164 |
| Pro-insulin^ (pmol/L) | 27.8 ± 3.9 | 1337 ± 130 | <0.0001 |
| C-Peptide^ (ng/ml) | 2.4 ± 0.2 | 6.4 ± 0.4 | <0.0001 |
| Amylin^ (active, pg/ml) | 45.6 ± 8.1 | 288.6 ± 25.6 | <0.0001 |
| Leptin^ (ng/ml) | 6.8 ± 0.9 | 41.0 ± 6.3 | <0.0001 |
| GIP^ (pg/ml) | 223.1 ± 20.0 | 218.6 ± 51.3 | 0.9372 |
| Glucagon^ (pg/ml) | 25.8 ± 2.5 | 37.8 ± 4.6 | 0.0388 |
| PP^ (pg/ml) | 26.1± 3.9 | 34.6 ± 8.0 | 0.3599 |
| PYY^ (pg/ml) | 32.8 ± 3.0 | 106.3 ± 13.8 | <0.0001 |
| Adiponectin^ (ng/ml) | 2.58 ± 0.09 | 1.85 ± 0.05 | <0.0001 |
| Clusterin (µg/ml) | 8.6 ± 0.2 | 12.0 ± 0.4 | <0.0001 |
| KIM-1 (pg/ml) | 294.0 ± 7.0 | 641.8 ± 94 | 0.0024 |
| NGAL (ng/ml) | 43.7 ± 2.4 | 66.1 ± 4.3 | 0.0005 |
| MCP1 (ng/ml) | 1.19 ± 0.09 | 1.28 ± 0.12 | 0.1497 |
| TNFa (pg/ml) | 29.4 ± 3.4 | 12.6 ± 2.9 | 0.0022 |
| IL-1b (pg/ml) | 39.1 ± 6.6 | 121.4 ± 26.7 | 0.0098 |
| TGFb (ng/ml) | 75.1 ± 1.5 | 70.0 ± 3.4 | 0.1934 |
| TIMP1 (ng/ml) | 3.33 ± 0.47 | 4.21 ± 0.34 | 0.1524 |

ZSF1 obese male rats at the age of 20 weeks exhibited dramatically elevated body weight (38% increase vs. lean group, p<0.0001), significantly increased blood glucose (1.6 fold, p=0.0060), serum insulin (14.7 fold, p=0.0019), cholesterol (2.9 fold, p<0.0001) and triglyceride (15 fold, p<0.0001) levels relative to ZSF-1 lean littermate controls (**Figures 1A**-**1E**, respectively). Significant decrease in serum adiponectin from 2.6 ± 0.09 µg/ml in ZSF1-lean serum to 1.9 ± 0.05 µg/ml in ZSF1-obese serum (p<0.0001) (**Figure 1F**) indicated deposition of newly formed visceral fat and served as a reliable obesity biomarker.

### Example 16: ZSF1 obese rats demonstrate pancreatic and renal disfunction

Since the array of biomarker changes relevant to the progression and establishment of cardiomatabolic syndrome in humans includes type 2 diabetes and renal impairment, the systemic levels of major pancreatic and kidney injury biomarkers in ZSH1-obese rats were evaluated and compared with their lean littermates (**Table 7)**. ZSF1 obese male rats at the age of 20 weeks demonstrated pancreatic disfunction significantly as shown by 2.7 folds elevated systemic C-peptide (**Figure 2A**), 47 folds increased proinsulin (**Figure 2B**), 6.3 folds raised serum active amylin (**Figure 2C**) and moderately, but significantly elevated glucagon (37.8 ± 4.6 in ZFF1 obese vs 25.8 ± 2.5 pg/ml in ZSF1 lean littermates, p=0.0388; **Figure 2D**). Impaired renal function in the ZSF1 obese cohort was characterized by significantly increased serum kidney injury markers, NGAL (1.5 folds vs ZSF1 lean, **Figure 2E**), KIM-1 (2.2 folds vs ZSF1 lean, **Figure 2F**) and clusterin (1.4 folds vs ZSF1 lean, **Figure 2G**).

### Example 17: ZSF1 obese rats exhibit diastolic dysfunction and reduced exercise capacity

The complete results of invasive hemodynamic assessment, echocardiography and exercise capacity of 20-21-week-old ZSF1 obese and lean male rats are presented in **Table 8.**

**Table 8: Invasive hemodynamic assessment, echocardiography and exercise capacity of 20-21-week-old ZSF1 obese and lean male rats**

| **Biomarker** | **Physical parameters (Mean ± SEM)** | | **P value** |
|---|---|---|---|
| | **ZSF1 Lean** | **ZSF1 OB** | |
| Heart/Brain Weight (ratio) | 0.68 ± 0.03 | 0.81 ± 0.01 | 0.0070 |
| Heart Rate (bpm) | 371.2 ± 9.5 | 292.8 ± 4.1 | <0.0001 |
| Diameter (s) (mm) | 3.59 ± 0.18 | 4.37 ± 0.34 | 0.053 |
| Diameter (d) (mm) | 8.0 ± 0.13 | 9.21 ± 0.28 | 0.0011 |
| Volume (s) (µl) | 56.07 ± 6.29 | 91.23 ± 14.71 | 0.0383 |
| Volume (d) (µl) | 345.8 ± 11.94 | 474.8 ± 29.16 | 0.0009 |
| Stroke Volume (µl) | 289.7 ± 10.0 | 383.5 ± 18.65 | 0.0005 |
| Ejection Fraction (%) | 83.93 ± 1.6 | 81.45 ± 2.32 | 0.3839 |
| Fractional Shortening (%) | 50.03 ± 1.97 | 52.89 ± 2.56 | 0.4919 |
| Cardiac Output (ml/min) | 107.4 ± 4.3 | 112 ± 5,1 | 0.4990 |
| LV Mass (mg) | 1274 ± 124.2 | 1445 ± 41.1 | 0.2384 |
| LV Mass Cor (mg) | 1019 ± 99.4 | 1156 ± 32.9 | 0.2384 |
| IVRT (ms) | 20.12 ± 0.55 | 24.89 ± 0.67 | <0.0001 |
| EDP (mmHg) | 1.808 ± 1.116 | 3.445 ± 1.342 | 0.3669 |
| Tau (ms) | 6.83 ± 0.33 | 9.53 ± 46 | 0.0004 |
| E/E' (Ratio) | 16.25 ± 1.07 | 25.07 ± 2.65 | 0.0064 |
| Distance (m) | 394.9 ± 27.9 | 194.0 ± 6.5 | <0.0001 |
| Time to Exhaustion (min) | 27.13 ± 1.34 | 17.75 ± 0.53 | <0.0001 |
| Peak VO2 (mg.kg-1.hr-1) | 3541 ± 130 | 2927 ± 33.4 | 0.0004 |
| Respiratory Exchange (Ratio) | 0.993 ± 0.017 | 0.9583 ± 0.015 | 0.1452 |

The heart to brain weight ratio was significantly higher in ZSF1 obese rats compared to ZSF1 lean (**Figure 3A**). Invasive hemodynamic assessment at 21 weeks under ketamine/diazepam anesthesia revealed an increased relaxation constant *tau* (**Figure 3B**). Echocardiography at 20 weeks under isoflurane anesthesia revealed that ZSF1 obese rats exhibit a significant increase in heart rate (**Figure 3C**), E/E' (**Figure 3D**) and IVRT (**Figure 3E**) while maintaining a normal ejection fraction (**Figure 3F**). It was further demonstrated that ZSF-1 obese rats exhibit a limited exercise capacity with significantly shorter distance (**Figure 3G**), shorter times to exhaustion (**Figure 3H**), and lower peak VO₂ (**Figure 3I**) following treadmill challenge.

### Example 18: ZSF1 obese rats exhibit changes in biomarkers of cardiovascular function

To evaluate the translational value of ZSF1 obese rat model for human cardiometabolic syndrome, the major systemic cardiovascular diseases biomarkers were profiled (**Table 9,** **Figures 4A-4F**) and compared to the results with the gene expression of the same markers in the left heart of 20-week-old ZSF1 obese and lean male rats.

**Table 9: Cardiovascular biomarkers in circulation of 20-week-old male ZSF1 obese and lean rats**

| **Biomarker** | **Serum/plasma^ concentration (Mean ± SEM)** | | **P value** |
|---|---|---|---|
| | **ZSF1 Lean** | **ZSF1 OB** | |
| Follistatin-like 1 (ng/ml) | 8.11 ± 0.18 | 6.92 ± 0.17 | 0.0002 |
| Osteopontin (ng/ml) | 0.96 ± 0.02 | 0.96 ± 0.03 | 0.9845 |
| GDF15^ | 62.0± 1.8 | 161.9 ± 17.3 | <0.0001 |
| BNP^ (pg/ml) | 27.1 ± 3.4 | 34.7 ± 5.4 | 0.2512 |
| sE-Selectin^ (ng/ml) | 98.6 ± 5.4 | 93.8 ± 3.4 | 0.4497 |
| FABP3 (pg/ml) | 64.8 ± 6.15 | 185.9 ± 32.36 | 0.0025 |
| FABP4 (ng/ml) | 42.41 ± 7.34 | 56.54 ± 7.63 | 0.2030 |
| FABP5 (ng/ml) | 11.28 ± 0.52 | 18.84 ± 1.16 | <0.0001 |
| Aldosterone (ng/ml) | 0.708 ± 0.081 | 1.658 ± 0.223 | 0.0010 |
| NT-proANP (ng/ml) | 9.67 ± 0.75 | 9.17 ± 0.92 | 0.6768 |
| NT-proBNP (pg/ml) | 99.18 ± 12.55 | 34.91 ± 3.39 | 0.0002 |
| MYBPC3 | 15.32 ± 2.85 | 9.12 ± 1.79 | 0.0864 |
| IL-16 (pg/ml) | 55.4 ± 2.26 | 418.3 ± 50.3 | <0.0001 |
| ST2 (pg/ml) | 435.9 ± 18.8 | 711.7 ± 29.4 | <0.0001 |
| Endothelin-1 (ET1) (pg/ml) | 1.48 ± 0.09 | 1.9 ± 0.19 | 0.0686 |
| FLT1/VEGFR1 (pg/ml) | 16.9 ± 1.22 | 19.32 ± 2.15 | 0.3447 |

The array of systemic changes in CVD-related biomarkers included increased blood levels of the hypertension marker aldosterone (**Figure 4A**), elevated levels of the biomarker of heart pathology FABP3 (**Figure 4B**), and significantly increased vascular markers IL-16 (**Figure 4C**) and ST2 (**Figure 4D**) in serum. Systemic concentration of NT-proBNP (NPPB) (**Figure 4E**) in ZSF1 obese rat was significantly lower vs. lean cohort, and osteopontin (OPN) (**Figure 4F**) levels in circulation were not different between obese and lean ZSF1. The gene expression of above markers in left heart was not different between obese and lean ZSF1 for FABP3, IL-16, IL-1RL1 (data not shown) and NPPB (**Figure 4G**), whereas the local SPP1 mRNA expression (osteopontin gene) in both left atria (LA) and left ventricle (LV) was significantly elevated in ZSF1 obese rats (**Figure 4H**).

### Example 19: Obesity-related gene expression changes in the left heart of ZSF1 rats

To characterize the transcriptional effects of obesity and metabolic disfunction on expression of heart tissue specific/abundant genes in left heart, RNA-seq was performed on left ventricle (LV) isolated from 20-week-old male ZSF1 obese and lean males. Comparison of heart abundant protein coding gene expression levels in left ventricle biopsies (LV) from ZSF1 lean and obese donors revealed a relatively small number of genotype-driven gene expression changes in heart-abundant protein coding genes: 56 genes demonstrated significantly increased (FC>1.5, p<0.05, **Table 10**) expression and the expression of 48 genes was significantly decreased (FC<0.67, p<0.05, **Table 11**) in obese cohort vs lean.

**Table 10: Heart abundant tissue gene expression increase in LV of ZSF1 obese vs lean group**

| **Gene** | **Log2 FC Increase** | **BH P value** | **Gene** | **Log2 FC Increase** | **BH P value** |
|---|---|---|---|---|---|
| Acaa2 | 0.925244 | 8.74E-44 | Lpar3 | 1.088838 | 1.58E-21 |
| Acadm | 0.623386 | 2.23E-21 | Lrp4 | 0.746815 | 5.26E-21 |
| Acot2 | 1.407497 | 5.41E-60 | Myh7 | 1.387513 | 3.22E-14 |
| Acox1 | 0.612524 | 2.40E-38 | Ncam1 | 1.067203 | 7.08E-11 |
| Adrala | 0.71756 | 0.000101 | Nppa | 0.713072 | 0.0066 |
| Alpk2 | 0.626377 | 3.55E-16 | Nrap | 0.713035 | 2.75E-35 |
| Alpk3 | 0.621277 | 9.72E-26 | Ntn1 | 0.67961 | 1.05E-14 |
| Ankrd23 | 1.166162 | 2.51E-13 | Pank1 | 0.585723 | 3.36E-20 |
| Aqp7 | 0.812687 | 1.77E-18 | Pcdh20 | 0.680139 | 0.012131 |
| Cadps | 0.710136 | 2.33E-09 | Pcyox1 | 0.601507 | 4.81E-19 |
| Corin | 0.716298 | 1.73E-20 | Pde3a | 0.746655 | 2.87E-08 |
| Cptla | 0.744796 | 8.84E-18 | Pde7b | 0.618594 | 1.51E-05 |
| Csdc2 | 0.616815 | 5.22E-10 | Pdk4 | 2.056103 | 2.27E-19 |
| Decr1 | 1.04417 | 2.83E-47 | Pdzd2 | 0.664067 | 2.16E-06 |
| Dusp26 | 0.762459 | 9.69E-05 | Plekha5 | 0.989596 | 5.11E-15 |
| Ech1 | 0.836496 | 2.03E-37 | Postn | 1.02457 | 0.000134 |
| Eci1 | 0.776771 | 3.66E-19 | Ppcs | 0.590606 | 3.78E-13 |
| Ehhadh | 0.614765 | 1.37E-10 | Ryr2 | 0.586292 | 1.69E-05 |
| Eya4 | 0.645467 | 7.92E-05 | Sema6c | 0.940007 | 2.26E-13 |
| Fhl1 | 0.746967 | 0.000126 | Slc16a1 | 0.599788 | 8.36E-21 |
| Fkbp5 | 1.020574 | 5.82E-10 | Sorbs1 | 0.61142 | 2.33E-06 |
| Gatsl2 | 0.990934 | 1.12E-11 | Sorbs2 | 0.691732 | 3.48E-18 |
| Grip2 | 0.813704 | 1.81E-06 | Sphkap | 0.948305 | 1.25E-13 |
| Hadha | 0.597629 | 6.57E-22 | Synpo | 0.682884 | 5.58E-23 |
| Hadhb | 0.588585 | 7.77E-28 | Sypl2 | 1.574139 | 3.43E-11 |
| Hcn4 | 1.787693 | 6.89E-11 | Vwa8 | 0.854878 | 9.36E-12 |
| Hipk2 | 1.018523 | 0.000114 | Xirp2 | 0.95803 | 3.01E-14 |
| Kcna4 | 0.819119 | 1.56E-08 | Zbtb25 | 0.64347 | 5.25E-05 |

**Table 11: Heart abundant tissue gene expression decrease in LV of ZSF1 obese vs lean group**

| **Gene** | **Log2 FC Decrease** | **BH P value** | **Gene** | **Log2 FC Decrease** | **BH P value** |
|---|---|---|---|---|---|
| Ace2 | -0.65349 | 1.51E-07 | Mrp133 | -6.523 | 1.93E-09 |
| Alkbh7 | -0.66233 | 1.11E-07 | Myct1 | -0.65596 | 1.04E-06 |
| Apln | -0.98546 | 1.93E-17 | Myh6 | -0.83876 | 5.9E-08 |
| Apold1 | -1.29498 | 2.45E-08 | Myl2 | -3.4613 | 1.48E-35 |
| Atpla2 | -0.6179 | 1.36E-17 | Myo16 | -0.91175 | 5.52E-05 |
| Atp5i | -1.03852 | 0.000927 | Myoz1 | -0.73592 | 0.003804 |
| Aurka | -0.62431 | 0.000256 | Ndufa3 | -1.21741 | 0.000483 |
| Btg2 | -1.15314 | 4.11E-08 | Nes | -1.04319 | 2.61E-30 |
| C7 | -0.60483 | 0.000167 | Ngf | -0.58577 | 0.001143 |
| Cdca7l | -0.66148 | 0.000639 | Nr4a2 | -1.2504 | 3.21E-10 |
| Cdh5 | -0.72207 | 3.41E-20 | Nrep | -0.94786 | 2.08E-10 |
| Cenpn | -0.6516 | 0.001388 | Omd | -0.59348 | 6.99E-05 |
| Cox7a2 | -0.60752 | 1.33E-06 | Pcdh12 | -0.9382 | 1.31E-15 |
| Csgalnact1 | -0.60155 | 2.97E-06 | Pet100 | -0.59974 | 0.000045 |
| Cxcl1 | -0.97868 | 0.022871 | Pfkfb2 | -0.74672 | 5.18E-11 |
| Egr2 | -2.0947 | 2.13E-15 | Pomp | -1.989 | 0.000193 |
| Gcat | -0.62848 | 3.12E-05 | Slc39a8 | -1.00884 | 5.57E-14 |
| Gimap8 | -0.7251 | 1.45E-12 | Slirp | -0.61681 | 1.13E-05 |
| Hspe1 | -0.82115 | 0.024196 | Tcf15 | -1.00965 | 1.49E-12 |
| Lgals3bp | -0.62544 | 1.84E-14 | Tmem88 | -0.80065 | 1.14E-12 |
| Lin9 | -0.62486 | 0.000165 | Tomm7 | -0.79847 | 2.86E-07 |
| Ltc4s | -1.10115 | 0.00606 | Usmg5 | -1.49256 | 0.010782 |
| Magohb | -0.61692 | 0.000599 | Vtn | -0.64253 | 4.85E-22 |
| Maob | -0.76571 | 2.23E-11 | Mlf1 | -0.72835 | 5.41E-18 |
| Mlf1 | -0.72835 | 5.41E-18 | Mrp133 | -6.523 | 1.93E-09 |

Notably, the IPA pathway enrichment analysis revealed (**Figure 5A**) that most of the gene expression changes in LV of ZSF 1-obese rats belong to fatty acid and branching amino acid metabolism pathways or to the cardiac signaling pathways (**Figure 5A**). The increase in ACADM, EHHADH, HADHA and HADHB gene expression was shared by both fatty acid and branching amino acid metabolism pathways. Altered ACAA2, ACOT2, ACSL6, ECI1, BDH1, IDI1 and HMGCS2 gene expression was fatty acid metabolism specific and altered expression of MAOA, MAOB, DUSP26, PHGDH and PSAT1 in LV was signatorial for the branching amino acid metabolism pathway (**Figure 5B**). The MYL2 and MYH6 gene expression decrease together with increased RYR2, HCN4, CORIN, NPPA, ERBB2 and MYH7 were the gene expression changes affecting hypertrophic and/or dilated cardiomyopathy.

Together, Examples 15-19 provide a compelling case that ZSF-1 obese rats exhibit multiple features of human cardiometabolic syndrome: pathological changes in renal, metabolic and CVD circulating biomarkers, obesity-driven changes in expression of heart abundant genes, cardiovascular disfunction with preserved ejection fraction (by hemodynamic assessment and echocardiography), and decreased exercise capacity.

The systemic levels of pancreas-related biomarkers in ZSF1-obese rats demonstrated pancreatic beta-cells disfunction as significantly increased serum C-peptide, proinsulin, glucagon and amylin (**Figures 2A-2D**) were observed. Renal disfunction is a part of CMD, and significantly increased systemic renal injury biomarkers KIM1, NGAL and clasterin (**Figures 2E-2G**) confirmed the presence of diabetic nephropathy and kidney damage in 20-week-old ZSF1-obese male rats.

Biomarkers are a powerful diagnostic and/or prognostic tool for a variety of CVD in humans. The milieu of systemic protein and local molecular cardiovascular biomarker changes (left ventricle of the heart) triggered by the development of CMD in ZSF1-obese rats was studied and the translational value of the observations evaluated. Notably, among the array of systemic changes there was a significant increase of aldosterone (**Figure 4A**) together with significant abrogation of heart abundant cardiomyocyte secreted protein NT-proBNP (**Figure 4E**) in circulation. There is a strong association of aldosterone increase with hypertension, obesity, chronic kidney disease, metabolic syndrome, high triglycerides, concentric left ventricular hypertrophy, atrial fibrillation and reverse correlation between aldosterone and NT-proBNP. During the last decade several scientific reports from human populational studies demonstrated that serum NT-proBNP concentrations were relatively lower in overweight and obese patients, suggesting a link between the heart and adipose tissue distribution mediated through natriuretic peptides. The observation that human subcutaneous adipose tissue from obese subjects with T2D exhibits markedly increased NP receptors expression leading to the higher clearance of NT-proBNP from the circulation could be a reasonable explanation of significantly decreased serum NT-proBNP without NPPB mRNA expression changes in left heart of ZSF1-obese rats (**Figure 4**).

The FABP3 (fatty-acid-binding protein 3) is abundantly expressed in cardiomyocyte cytoplasm, its circulation levels are positively correlated with left ventricle mass index and is proposed to be an early biomarker of myocardial injury in humans. The ST2, circulating protein marker of cardiomyocyte stress and fibrosis, that increases in patients across a wide spectrum of cardiovascular diseases, is now recommended by American College of Cardiology Foundation and American Heart Association joint guidelines for additive risk stratification in patients with heart failure. Chronic inflammation contributes to cardiac fibrosis, and systemic interleukin-16 (IL-16) levels are specifically elevated in HFpEF patients (compared with HFrEF and controls) with a significant association between serum IL-16 and indices (LAVI, E/E' and DWS) of LV diastolic dysfunction. In concert with the published results from human studies serum FABP3, ST2 and IL-16 protein concentrations were significantly elevated in 20-week-old ZSF1-obese male rats (**Figures 4B-4D****)** and supported translational value of this preclinical model of human CMS.

The Examples also demonstrate that physical parameters of heart failure with preserved ejection fraction (HFpEF)-specific heart disfunction coincided with pathological changes in CVD circulating biomarkers. Among the biomarkers of HF progression is osteopontin (OPN, abundant kidney protein majorly synthesized in the loop of Henle) that is expressed at medium level in heart tissue by endothelial cells, cardiomyocytes and fibroblasts.

Based on the transcriptome analysis (https://www.proteinatlas.org/humanproteome/tissue/heart) approximately 60% (n=12224 out of 19613) of protein coding genes are expressed in the heart tissue. Two hundred of these genes show an elevated expression in heart compared to other tissue types, and most of the corresponding proteins (localized in the cytoplasm and in the sarcomeres) are involved in muscle contraction, ion transport and ATPase activity. The comparative RNAseq analysis (ZSF1-obese vs lean) of rat LV heart abundant genes described here revealed the dramatic changes in cardiomyocyte, fibroblast and vascular cells gene expression, i.e. significant increase in expression of 59 genes (**Table 10**) and significant decrease in expression of 48 genes (**Table 11**) in 20-week-old ZSF1-obese males.

In ZSF1 rat CMS model the obesity and impaired metabolism have also greatly increased CVD pathology by altering two major metabolic pathways in heart tissue (Figure 5B) - fatty acid (FADS) metabolism and branched- chain amino acid (BCAA) metabolism.

In general, cardiac metabolic homeostasis of fatty acid, glucose, ketone bodies and branched-amino acids are well-established through an intertwined regulatory network. Thus, gene expression of ACADM, EHHADH, HADHA and HADHB, that are essential enzymes involved in both fatty acid metabolism and BCAA catabolism, is greatly increased in ZSF1-obese LV heart tissue. It has been reported that acyl-CoA deficiency in human failing heart disrupts cardiac energy production and leads to cardiac lipotoxisity that in return impairs heart's ability to function and pump properly and ACAA2, ACADM, ACSL6, ACOT2, EHHADH, HADHA and HADHB are enzymes functionally involved in acyl-CoA metabolism. By human GWAS database analysis the mutations in HADHA and HADHB are associated with familial hypertrophic cardiomyopathy and mutations in ACADM, ACAA2 genes are associated with mitochondrial fatty acid β-oxidation. Several enzymes (ACADM, ACSL6, EHHADH and HMGCS2) are regulated by proliferator activated receptors (PPARs), that manipulate the fuel supply and substrate and modulate the heart failure progression. IPA and MeSH based cardiac signaling and disease analysis demonstrated that significant changes in handful of cardiomyocyte genes (decreased MYL2 and MYH6 and increased RYR2, HCN4, CORIN, NPPA, ERBB2 and MYH7)) were strongly associated with dilated cardiomyopathy, LV hypertrophy and heart failure, and are similar to those observed in human genetic studies aimed to elucidate the genetic basis of cardiomyopathy and heart failure. Hence, the novel RNAseq results in the Examples here demonstrated the array of expression changes in LV heart-abundant genes in ZSF1-obese rats, mainly those involved in fatty acid metabolism, branching amino acid metabolism, hypertrophy, cardiomyopathy and heart failure.

### Example 20: ZSF1 obese rats treated with GDF15 exhibit metabolism improvement by changes in systemic parameters and biomarkers of obesity and metabolism impairment

For the duration of FC-GDF15 or Vehicle treatment (see Example 2) both cohorts of ZSF1 obese rat were monitored weekly for the food intake and body weight and bi-weekly for blood levels of cholesterol, triglycerides, insulin and glucose (**Figure 6**). Within first two weeks of the FC-GDF15 treatment, significant and sustained decrease (to the end of the study) in body weight (**Figure 6A**), food intake (**Figure 6B**), blood triglycerides (**Figure 6C**) and blood glucose (**Figure 6D**) was observed and documented. Blood cholesterol was 20-30% lower in FC-GDF15 treated cohort of ZSF1 obese rats (vs Vehicle group) from week 1 to week 12 of the treatment, but such decrease did not achieve significance at any tested time point of the study (**Figure 6E**)**.** At the same time blood insulin levels significantly increased at weeks 9 and 12 of FC-hGDF15 treatment (**Figure 6F**) compared to the vehicle group. At the end of 12-week-long FC-GDF15 treatment circulating adiponectin concentration was significantly increased (**Figure 6G**). At the same time the endogenous rat GDF15 level in serum of FC-hGDF15 treated and Vehicle treated ZSF1 obese rats remained similar (**Figure 6H**).

### Example 21: GDF15 treatment of ZSF1 obese rats led to the significant positive changes in systemic levels of cardiovascular biomarkers

The systemic levels of cardiovascular biomarkers in ZSF1 obese rats were evaluated at the end of the study and are presented in **Table 12.**

**Table 12: Effect of GDF15 treatment on systemic levels of cardiovascular markers**

| **Biomarker** | **Serum concentration at wk 12 of FC-GDF15 treatment (Mean ± SEM)** | | **P value** |
|---|---|---|---|
| | **Vehicle group** | **hGDF15 group** | |
| Adiponectin (µg/ml) | 3.15 ± 0.15 | 3.95 ± 0.17 | 0.0015 |
| Aldosterone (pg/ml) | 261.5 ± 45.3 | 237.1 ± 45.8 | 0.7118 |
| BNP (pg/ml) | 4.14 ± 1.16 | 4.67 ± 1.66 | 0.8204 |
| sE-Selectin (ng/ml) | 245.6 ± 12.6 | 146.0 ± 12.0 | <0.0001 |
| FABP3 (ng/ml) | 7.52 ± 1.53 | 5.93 ± 2.28 | 0.5549 |
| sICAM (ng/ml) | 29.97 ± 2.01 | 15.72 ± 2.04 | <0.0001 |
| MCP1 (ng/ml) | 4.16 ± 0.24 | 3.52± 0.22 | 0.0683 |
| Myl3 (pg/ml) | 154.1 ± 14.41 | 99.9 ± 22.6 | 0.0458 |
| NT-proANP (ng/ml) | 20.44 ± 1.30 | 18.08 ± 1.54 | 0.2490 |
| NT-proBNP (ng/ml) | 12.65 ± 1.98 | 15.90 ± 2.53 | 0.3148 |
| Osteopontin (ng/ml) | 2.09 ± 0.14 | 1.70 ± 0.12 | 0.0494 |
| TIMP1 (ng/ml) | 21.04 ± 2.14 | 12.91 ± 2.08 | 0.01129 |
| VEGF (pg/ml) | 72.87 ± 6.76 | 25.0 ± 10.63 | 0.0006 |
| vWF (ng/ml) | 186.9 ± 9.83 | 125.1 ± 15.2 | 0.0016 |
| GDF15 (pg/ml) | 392.6 ± 33.3 | 443.2 ± 84.2 | 0.5512 |

Following 12 weeks of FC-hGDF15 administration significant positive changes in the array of cardiovascular biomarkers in circulation was observed. Thus, systemic markers of cardiac injury vWF (**Figure 7A**) and Myl3 (**Figure 7B**) were 30-50% lower (p<0.05) in FC-hGDF15 treated group. Heart failure associated osteopontin (**Figure 7C**), markers of vascular injury E-Selectin (**Figure 7D**), sICAM (**Figure 7E**), and VEGF (**Figure 7F**) and marker of cardiovascular fibrosis TIMP-1 (**Figure 7G**) were significantly lower in serum/plasma of GDF15-treated vs. Vehicle-treated ZSF1 obese rats ((p<0.05). At the same time the other systemic biomarkers of CVD, such as NT-proBNP, NT-proANP, BNP, FABP3, MCP1 and aldosterone were not systemically changed by 12-week-long FC-hGDF15 treatment (**Table 12**).

### Example 22: GDF15 therapeutic approach effected echography parameters of left heart function and improved exercise capacity in ZSF1 obese rats

When the ZSF1 obese rats were characterized, it was demonstrated that at the age of 20 weeks males exhibit diastolic dysfunction and decreased exercise capacity. Twelve weeks of FC-hGDF15 treatment of ZSF1 obese males resulted in significantly decreased heart to brain weight ratio (by echocardiography, **Table 13,** **Figure 8**), LV mass, cardiac output, stroke volume and % ejection fraction echocardiographic parameters, when compared to ZSF1 obese rats treated with Vehicle (**Figures 8A-8E**). GDF15 treatment had no effect on heart rate (**Figure 8F**).

**Table 13: Effect of 12 week-long hGDF15 treatment on parameters of invasive hemodynamic assessment, echocardiography and exercise capacity of ZSF1 obese male rats**

| **Biomarker** | **Physical parameters (Mean ± SEM)** | | **P value** |
|---|---|---|---|
| | **Vehicle group** | **FC-GDF15 group** | |
| Heart/Brain Weight (ratio) | 0.907 ± 0.01 | 0.765 ± 0.01 | <0.0001 |
| Heart Rate (bpm) | 294.0 ± 5.8 | 284.9 ± 6.4 | 0.3024 |
| Diameter (s) (mm) | 5.35 ± 0.23 | 5.63 ± 0.13 | 0.3458 |
| Diameter (d) (mm) | 9.56 ± 0.15 | 9.15 ± 0.16 | 0.0711 |
| Volume (s) (µl) | 143.7 ± 14.06 | 156.4 ± 8.42 | 0.4722 |
| Volume (d) (µl) | 512.8 ± 17.93 | 467.8 ± 17.87 | 0.0914 |
| Stroke Volume (µl) | 369.1 ± 10.04 | 311.4 ± 13.02 | 0.0015 |
| Ejection Fraction (%) | 72.59 ± 1.98 | 66.58 ± 1.23 | 0.0225 |
| Fractional Shortening (%) | 44.17 ± 1.75 | 38.63 ± 1.01 | 0.0167 |
| Cardiac Output (ml/min) | 108.7 ± 3.95 | 88.49 ± 3.84 | 0.0013 |
| LV Mass (mg) | 1657 ± 51.83 | 1317 ± 62.41 | 0.0003 |
| LV Mass Cor (mg) | 1326 ± 41.46 | 1054 ± 49.93 | 0.0003 |
| IVRT (ms) | 25.72 ± 0.65 | 28.52 ± 1.19 | 0.0399 |
| EDP (mmHg) | 15.94 ± 0.63 | 16.27± 1.02 | 0.7733 |
| Tau (ms) | 13.79 ± 0.57 | 13.86 ± 0.87 | 0.9414 |
| E/E' (Ratio) | 18.49 ± 0.75 | 16.98 ± 0.73 | 0.1697 |
| Distance (m) | 114.9 ± 7.95 | 151.8 ± 12.32 | 0.0173 |
| Time to Exhaustion (min) | 9.98 ± 0.57 | 12.37 ± 0.7 | 0.0160 |
| Peak VO2 (mg.kg-1.hr-1) | 3093 ± 74.3 | 3122 ± 123.2 | 0.8331 |
| Respiratory Exchange (Ratio) | 1.008 ± 0.018 | 0.9949 ± 0.015 | 0.5998 |

After the twelve weeks of treatment, when exposed to a treadmill challenge, the FC-hGDF15 group demonstrated improved exercise capacity (**Table 13**) by exhibiting significantly longer running time (**Figure 8G**) and running distance (**Figure 8H**) vs. Vehicle group. However, peak VO2 (a measure of aerobic fitness) and the respiratory exchange ratio (RER, a fatigue measure) were not significantly different between groups (**Table 13**) suggesting that both groups were run to the same level of exhaustion.

In Examples 20-22, ZSF1-obese rats were treated with a pharmacological dose of Fc-GDF15 for 12 weeks and compared the outcome with the ZSF1-obese cohort treated with Vehicle. Twelve-week-long GDF15 treatment resulted in significantly decreased body weight and food intake, confirming its capacity to mediate aversive dietary response. Fc fusion GDF15 molecule improved metabolic parameters (decreased blood glucose and triglycerides) in ZSF1-obese rats (**Figure 6A-6F**). Serum adiponectin in GDF15-treated ZSF1-obese cohort was significantly increased. Adiponectin is secreted majorly by adipocytes, but also by skeletal and cardiac myocytes and endothelial cells. Its reduction plays a central role in CMS, since adiponectin is positively associated with insulin sensitivity, anti-atherogenic, and anti-inflammatory properties, and according to the numerous epidemiological studies hypoadiponectinemia (adiponectin deficiency) is additionally associated with such cardiovascular diseases as hypertension, CAD and left ventricular hypertrophy. While circulating adiponectin was decreased by 30% in ZSF1-obese vs lean (**Figure 1F**), the GDF15 treatment led to its significant systemic increase (29%, **Figure 6G**) vs. ZSF1-obese cohort treated with Vehicle. Even though, GDF15 treatment did not lead to the systemic changes in aldosterone, NT-proBNP and FABP3 in ZSF1-obese rats, the novel and exploratory systemic protein markers of cardiac injury vWF and Myl3, HF-associated osteopontin, markers of vascular injury E-Selectin, sICAM, and VEGF and cardiovascular fibrosis marker TIMP-1 were significantly lower in GDF15-treated obese rats vs. Vehicle cohort (**Figure 7**). In addition to the positive changes in the array of systemic cardiometabolic biomarkers, 12-week-long Fc-GDF 15 therapy led to the considerably decreased heart weight together with improved parameters of LV function (decreased LV mass, cardiac output and stroke volume, **Figure 8**) and exercise capacity (time to exhaustion and distance, **Figure 8**), when compared to Vehicle-treated ZSF1-obese littermates. In summary, ZSF1-obese rat males represent translational preclinical model of human CMS with established HFpEF. Furthermore, GDF15 treatment of ZSF1-obese rats demonstrated its cardioprotective therapeutic effect.

While the present invention has been described in terms of various embodiments, it is understood that variations and modifications will occur to those skilled in the art.

## Claims

1. A GDF15 molecule for use in a method of treating a heart condition in a subject, the method comprising administering the GDF15 molecule to the subject, wherein the heart condition is heart failure with preserved ejection fraction (HFpEF).

2. A GDF15 molecule for use in a method of treating a subject with cardiometabolic syndrome, the method comprising administering the GDF15 molecule to the subject.

3. The GDF15 molecule for use according to claim 2, wherein the subject is obese, has impaired metabolism, pancreatic dysfunction, hypertension, diastolic dysfunction, HFpEF, decreased exercise capacity, or renal dysfunction.

4. The GDF15 molecule for use according to claim 3, wherein the subject has a decreased heart weight, improved exercise capacity, or change in the level of adiponectin, sE-selectin, sICAM, Myl3, osteopontin, TIMP1, VEGF, or vWF, after treatment with the GDF15 molecule.

5. The GDF15 molecule for use according to claim 4, wherein the level of adiponectin is increased after treatment with the GDF15 molecule.

6. The GDF15 molecule for use according to claim 3 or 4, wherein the level of sE-selectin, sICAM, Myl3, osteopontin, TIMP1, VEGF, vWF, or any combination thereof is decreased after treatment with the GDF15 molecule.

7. The GDF15 molecule for use according to any one of claims 3-6, wherein the subject has decreased heart to brain weight ratio, left ventricle (LV) mass, cardiac output, stroke volume, or % ejection fraction echocardiographic, after treatment with the GDF15 molecule.

8. The GDF15 molecule for use according to any one of claims 1-7, wherein the GDF15 molecule is a fusion protein comprising a GDF15 region joined to an Fc region.

9. The GDF15 molecule for use according to claim 8, wherein the GDF15 region is joined to the Fc region via a linker.

10. The GDF15 molecule for use according to claim 8 or 9, wherein the GDF15 region comprises the amino acid sequence of SEQ ID NO: 6 and at least one mutation.

11. The GDF15 molecule for use according to claim 10, wherein at least one of the mutations is of the aspartate at position 5, preferably the aspartate at position 5 is mutated to glutamate.

12. The GDF15 molecule for use according to claim 10 or 11, wherein the GDF15 region further comprises a mutation of the asparagine at position 3, preferably the asparagine at position 3 is mutated to glutamine.

13. The GDF15 molecule for use according to any one of claims 9-12, wherein the linker is a (G₄S)ₙ or (G₄Q)ₙ linker, wherein n is greater than 0, preferably n is 1 or 2.

14. The GDF15 molecule for use according to any one of claims 8-13, wherein the Fc region comprises a charged pair mutation.

15. The GDF15 molecule for use according to any one of claims 8-14, wherein the Fc region comprises a truncated hinge region.

## Patentansprüche

1. Ein GDF15-Molekül zur Verwendung in einem Verfahren zur Behandlung eines Herzleidens in einem Patienten, wobei das Verfahren die Verabreichung des GDF15-Moleküls an den Patienten umfasst, wobei das Herzleiden eine Herzinsuffizienz mit erhaltener Auswurffraktion (HFpEF) ist.

2. Ein GDF15-Molekül zur Verwendung in einem Verfahren zur Behandlung eines Patienten mit kardiometabolischem Syndrom, wobei das Verfahren die Verabreichung des GDF15-Moleküls an den Patienten umfasst.

3. Das GDF15-Molekül zur Verwendung gemäß Anspruch 2, wobei der Patient fettleibig ist, einen gestörten Stoffwechsel, eine Pankreasdysfunktion, Hypertonie, eine diastolische Dysfunktion, HFpEF, eine verminderte körperliche Leistungsfähigkeit oder eine Nierenfunktionsstörung aufweist.

4. Das GDF15-Molekül zur Verwendung nach Anspruch 3, wobei der Patient nach der Behandlung mit dem GDF15-Molekül ein vermindertes Herzgewicht, eine verbesserte körperliche Leistungsfähigkeit oder eine Veränderung des Spiegels von Adiponectin, sE-Selectin, sICAM, Myl3, Osteopontin, TIMP1, VEGF oder vWF aufweist.

5. Das GDF15-Molekül zur Verwendung nach Anspruch 4, wobei der Adiponectinspiegel nach der Behandlung mit dem GDF15-Molekül erhöht ist.

6. Das GDF15-Molekül zur Verwendung nach Anspruch 3 oder 4, wobei der Spiegel von sE-Selectin, sICAM, Myl3, Osteopontin, TIMP1, VEGF, vWF oder einer beliebigen Kombination davon nach der Behandlung mit dem GDF15-Molekül verringert ist.

7. Das GDF15-Molekül zur Verwendung nach einem der Ansprüche 3 bis 6, wobei der Patient nach der Behandlung mit dem GDF15-Molekül ein verringertes Herz-Hirn-Gewichtsverhältnis, eine verringerte Masse des linken Ventrikels (LV), ein verringertes Herzzeitvolumen, ein verringertes Schlagvolumen oder eine verringerte echokardiographische prozentuale Auswurffraktion aufweist.

8. Das GDF15-Molekül zur Verwendung nach einem der Ansprüche 1-7, wobei das GDF15-Molekül ein Fusionsprotein ist, das eine GDF15-Region umfasst, die mit einer Fc-Region verbunden ist.

9. Das GDF15-Molekül zur Verwendung nach Anspruch 8, wobei die GDF15-Region über einen Linker mit der Fc-Region verbunden ist.

10. Das GDF15-Molekül zur Verwendung nach Anspruch 8 oder 9, wobei die GDF15-Region die Aminosäuresequenz von SEQ ID NO: 6 und mindestens eine Mutation umfasst.

11. Das GDF15-Molekül zur Verwendung nach Anspruch 10, wobei mindestens eine der Mutationen das Aspartat an Position 5 betrifft, vorzugsweise ist das Aspartat an Position 5 zu Glutamat mutiert.

12. Das GDF15-Molekül zur Verwendung nach Anspruch 10 oder 11, wobei die GDF15-Region ferner eine Mutation des Asparagins an Position 3 umfasst, vorzugsweise ist das Asparagin an Position 3 zu Glutamin mutiert.

13. Das GDF15-Molekül zur Verwendung nach einem der Ansprüche 9-12, wobei der Linker ein (G₄S)ₙ - oder (G₄ Q)ₙ-Linker ist, wobei n größer als 0 ist, vorzugsweise n gleich 1 oder 2 ist.

14. Das GDF15-Molekül zur Verwendung nach einem der Ansprüche 8-13, wobei die Fc-Region eine Mutation eines geladenen Paares umfasst.

15. Das GDF15-Molekül zur Verwendung nach einem der Ansprüche 8-14, wobei die Fc-Region eine verkürzte Scharnierregion umfasst.

## Revendications

1. Molécule GDF15 pour l'utilisation dans un procédé de traitement d'une affection cardiaque chez un sujet, le procédé comprenant l'administration de la molécule GDF15 au sujet, dans laquelle l'affection cardiaque est une insuffisance cardiaque avec fraction d'éjection préservée (HFpEF).

2. Molécule GDF15 pour l'utilisation dans un procédé de traitement d'un sujet présentant un syndrome cardiométabolique, le procédé comprenant une administration de la molécule GDF15 au sujet.

3. Molécule GDF15 pour l'utilisation selon la revendication 2, dans laquelle le sujet est obèse, présente un métabolisme altéré, un dysfonctionnement pancréatique, une hypertension, un dysfonctionnement diastolique, un HFpEF, une capacité réduite de faire de l'exercice, ou un dysfonctionnement rénal.

4. Molécule GDF15 pour l'utilisation selon la revendication 3, dans laquelle le sujet présente un poids réduit du cœur, une capacité améliorée de faire de l'exercice, ou un changement dans le niveau d'adiponectine, de sE-sélectine, de sICAM, de Myl3, de l'ostéopontine, de TIMP1, du VEGF, ou du vWF, après traitement avec la molécule GDF15.

5. Molécule GDF15 pour l'utilisation selon la revendication 4, dans laquelle le niveau d'adiponectine est accru après traitement avec la molécule GDF15.

6. Molécule GDF15 pour l'utilisation selon la revendication 3 ou 4, dans laquelle le niveau de sE-sélectine, de sICAM, de Myl3, de l'ostéopontine, de TIMP1, du VEGF, du vWF, ou de n'importe quelle combinaison de ceux-ci est réduit après traitement avec la molécule GDF15.

7. Molécule GDF15 pour l'utilisation selon l'une quelconque des revendications 3 à 6, dans laquelle le sujet présente un ratio pondéral cœur à cerveau, une masse du ventricule gauche (LV), un débit cardiaque, un volume d'éjection systolique, ou un % échocardiographique de fraction d'éjection réduits, après traitement avec la molécule GDF15.

8. Molécule GDF15 pour l'utilisation selon l'une quelconque des revendications 1 à 7, dans laquelle la molécule GDF15 est une protéine de fusion comprenant une région GDF15 jointe à une région Fc.

9. Molécule GDF15 pour l'utilisation selon la revendication 8, dans laquelle la région GDF15 est jointe à la région Fc par l'intermédiaire d'un lieur.

10. Molécule GDF15 pour l'utilisation selon la revendication 8 ou 9, dans laquelle la région GDF15 comprend la séquence d'acides aminés de SEQ ID n° : 6 et au moins une mutation.

11. Molécule GDF15 pour l'utilisation selon la revendication 10, dans laquelle au moins l'une des mutations est de l'aspartate à la position 5, préférablement l'aspartate à la position 5 est muté en glutamate.

12. Molécule GDF15 pour l'utilisation selon la revendication 10 ou 11, dans laquelle la région GDF15 comprend en outre une mutation de l'asparagine à la position 3, préférablement l'asparagine à la position 3 est mutée en glutamine.

13. Molécule GDF15 pour l'utilisation selon l'une quelconque des revendications 9 à 12, dans laquelle le lieur est un lieur (G₄S)ₙ ou (G₄Q)ₙ, dans laquelle n est supérieur à 0, préférablement n a la valeur de 1 ou 2.

14. Molécule GDF15 pour l'utilisation selon l'une quelconque des revendications 8 à 13, dans laquelle la région FC comprend une mutation de paire chargée.

15. Molécule GDF15 pour l'utilisation selon l'une quelconque des revendications 8 à 14, dans laquelle la région FC comprend une région charnière tronquée.
